# EUROPÄISCHE PATENTANMELDUNG

(11) **EP 2 343 276 A1**
(43) Veröffentlichungstag der Anmeldung: **13.07.2011**
(21) Anmeldenummer: 10184196.3
(22) Anmeldetag: 05.08.1998
(51) Int. Cl.: C07D 207/20, C07D 303/22, C07D 305/06, C07D 307/28, C07D 333/16, A01N 43/04, A01N 43/08, A01N 43/10, A01N 43/36

(54) **2-Benzoyl-cyclohexan-1,3-dione als Herbizide**

(30) Priorität: 07.08.1997 DE 19734164
(62) Teilanmeldung aus: 98942611.9
(71) Anmelder: BASF SE, 67056 Ludwigshafen (DE)
(72) Erfinder: Engel, Stefan, 65510 Idstein (DE); Rheinheimer, Joachim, 67063 Ludwigshafen (DE); Baumann, Ernst, 67346 Speyer (DE); von Deyn, Wolfgang, 67435 Neustadt (DE); Hill, Regina, 40589 Düsseldorf (DE); Mayer, Guido, 67161 Gönnheim (DE); Misslitz, Ulf, 67433 Neustadt (DE); Wagner, Oliver, 67433 Neustadt (DE); Witschel, Matthias, 67061 Ludwigshafen (DE); Otten, Martina, 85356 Freising (DE); Walter, Helmut, 67283 Obrigheim (DE); Westphalen, Karl-Otto, 67346 Speyer (DE)
(74) Vertreter: Büchel, Edwin

(57) **Zusammenfassung**

Die Erfindung betrifft substituierte 2-Benzoyl-cyclohexan-1,3-dione der Formel (I), in der Substituenten die in der Beschreibung angegebene Bedeutung haben, sowie deren landschaftlich brauchbaren Salze, Verfahren und Zwischenprodukte zur Herstellung von Verbindungen der Formel (I), Mittel, welche diese enthalten, sowie die Verwendung der Formel (I), Mittel, welche diese enthalten, sowie die Verwendung der Formel (I) und diese enthaltende Mittel zur Schadpflanzenbekämpfung.

## Beschreibung

Die vorliegende Erfindung betrifft substituierte 2-Benzoyl-cyclohexan-1,3-dione der Formel I 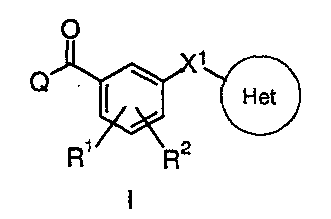 in der die Substituenten folgende Bedeutung haben:
R¹, R² Wasserstoff, Mercapto, Nitro, Halogen, Cyano, Rhodano, C₁-C₆-Alkyl, C₁-C₆-Halogenalkyl, C₁-C₆-Alkoxy, C₂-C₆-Alkenyl, C₂-C₆-Alkinyl, -OR³, -OCOR³, -OSO₂R³, -S(O)ₙR³, -SO₂OR³, -SO₂N(R³)₂, -NR³SO₂R³ oder -NR³COR³;
R³ Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/ oder eine bis drei der folgenden Gruppen tragen können:
   Hydroxy, Mercapto, Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
n 0, 1 oder 2;
Q ein gegebenenfalls substituierter, in 2-Stellung verknüpfter Cyclohexan-1,3-dion-Ring;
X¹ eine geradkettige oder verzweigte C₁-C₆-Alkylen-, eine C₂-C₆-Alkenylen- oder eine C₂-C₆-Alkinylenkette,die durch ein Heteroatom ausgewählt aus der Gruppe:
   Sauerstoff oder Schwefel
      unterbrochen ist und wobei die genannten Alkyl-, Alkenyl-oder Alkinylreste partiell halogeniert sein können und/ oder eine bis drei der folgenden Gruppen tragen können:

      -OR⁴, -OCOR⁴, -OCONHR⁴ oder -OSO₂R⁴;
R⁴ Wasserstoff, C₁-C₆-Alkyl, C₂-C₆-Alkenyl, C₂-C₆-Alkinyl, Phenyl, Phenyl-C₁-C₆-alkyl, wobei die genannten Alkyl-, Alkenyl oder Alkinylreste partiell oder vollständig halogeniert sein können und/ oder durch einen oder mehrere der folgenden Reste substituiert sein können:
   Hydroxy, Mercapto, Amino, Cyano; Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
Het eine drei- bis sechsgliedrige, teilweise oder vollständig gesättigte, heterocyclische Gruppe oder eine drei- bis sechsgliedrige heteroaromatische Gruppe mit bis zu drei Heteroatomen ausgewählt aus folgenden drei Gruppen:
   Stickstoff,
      Sauerstoff in Kombination mit mindestens einem Stickstoff oder
   Schwefel in Kombination mit mindestens einem Stickstoff,
   wobei die gennante heterocyclische oder heteroaromatische Gruppe partiell oder vollständig halogeniert sein kann und/oder durch R⁵ substituiert sein kann;
R⁵ Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkyl-carbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
   Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
sowie deren landwirtschaftlich brauchbaren Salze.

Außerdem betrifft die Erfindung Verfahren und Zwischenprodukte zur Herstellung von Verbindungen der Formel I, Mittel welche diese enthalten, sowie die Verwendung der Verbindungen der Formel I und diese enthaltende Mittel zur Schadpflanzenbekämpfung.

Aus der Literatur, beispielsweise aus EP-A 278 742, EP-A 298 680, EP-A 320 864 und WO 96/14285 sind 2-Benzoylcyclohexan-1,3-dione bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die erfindungsgemäßen 2-Benzoyl-cyclohexan-1,3-dione der Formel I sowie deren herbizide Wirkung gefunden.

Ferner wurden herbizide Mittel gefunden, die die Verbindungen I enthalten und eine sehr gute herbizide Wirkung besitzen. Darüber hinaus wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon erfaßt.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium. Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Hervorzuheben sind die erfindungsgemäßen Verbindungen der Formel I, wobei die Variable Q einen in 2-Stellung verknüpften Cyclohexan-1,3-dionring der Formel II darstellt, 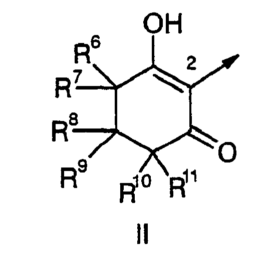 wobei II auch stellvertretend für die tautomeren Formeln II' und II" steht, 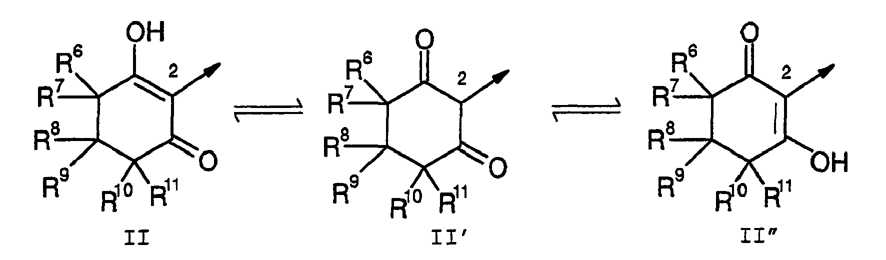 wobei
R⁶, R⁷, R⁹ und R¹¹ für Wasserstoff oder C₁-C₄-Alkyl stehen;
R⁸ für Wasserstoff, C₁-C₄-Alkyl oder C₃-C₄-Cycloalkyl steht, wobei die beiden letztgenannten Gruppen einen bis drei der folgenden Substituenten tragen können:
   Halogen, C₁-C₄-Alkylthio oder C₁-C₄-Alkoxy;
      oder
   für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch ein bis drei C₁-C₄-Alkylreste substituiert sein können;
R¹⁰ für Wasserstoff, C₁-C₄-Alkyl oder C₁-C₆-Alkoxycarbonyl steht;
   oder
R⁸ und R¹¹ gemeinsam eine π-Bindung oder einen drei- bis sechsgliedrigen carbocyclischen Ring bilden;
   oder

die CR⁸R⁹-Einheit durch C=O ersetzt sein kann.
Verfahren A:
Umsetzungen von Cyclohexan-1,3-dion der Formel II mit einer aktivierten Carbonsäure IIIa oder einer Carbonsäure IIIb die vorzugsweise in situ aktiviert wird, zu dem Acylierungsprodukt IV und anschließende Umlagerung zu den erfindungsgemäßen Verbindungen der Formel I. 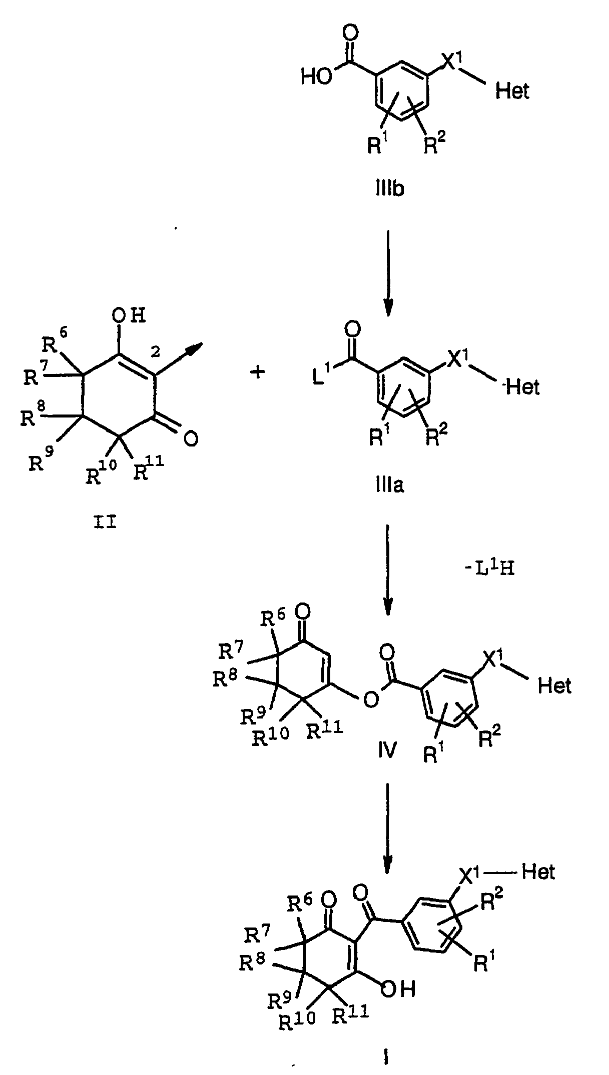
L¹ steht für eine nucleophil austauschbare Abgangsgruppe, wie Halogen z. B. Brom, Chlor, Hetaryl, z. B. Imidazolyl, Pyridyl, Carboxylat, z. B. Acetat, Trifluoracetat etc.

Die aktivierte Carbonsäure kann direkt eingesetzt werden, wie im Fall der Carbonsäurehalogenide oder in situ erzeugt werden, z. B. mit Dicyclohexylcarbodiimid, Triphenylphosphin/Azodicarbonsäureester, 2-Pyridindisulfid/.Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z. B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z. B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0 - 10 °C abzukühlen. Anschließend rührt man bei 20 - 100 °C, vorzugsweise bei 25 - 50 °C, bis die Umsetzung vollständig ist. Die Aufarbeitung erfolgt in üblicher Weise, z. B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel IV vorzugsweise durch Chromatographie gereinigt. Es ist aber auch möglich, den rohen Enolester der Formel IV ohne weitere Reinigung zur Umlagerung einzusetzen.

Die Umlagerung der Enolester der Formel IV zu den Verbindungen der Formel I erfolgt zweckmäßigerweise bei Temperaturen von 20 bis 40 °C in einem Lösungsmittel und in Gegenwart einer Base sowie gegebenenfalls in Gegenwart einer Cyanoverbindung.

Als Lösungsmittel können z. B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z. B. in einer Menge von 5 bis 15, vorzugsweise 10 Molprozent, bezogen auf den Ester, eingesetzt.

Besonders bevorzugt werden Alkalicarbonate, wie Kaliumcarbonat, in Acetonitril oder Dioxan eingesetzt.

Die Aufarbeitung kann in an sich bekannter Weise erfolgen. Das Reaktionsgemisch wird z. B. mit verdünnter Mineralsäure, wie 5 %ige Salzsäure oder Schwefelsäure, angesäuert, mit einem organischen Lösungsmittel, z. B. Methylenchlorid, Essigsäureethylester extrahiert. Der organische Extrakt kann mit 5 - 10 %iger Alkalicarbonatlösung, z. B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

Die Benzoesäuren der Formel III sind neu, 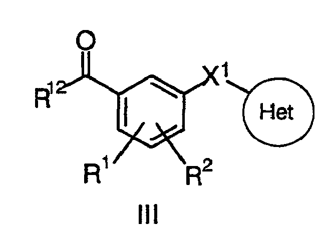 wobei die Variablen folgende Bedeutung haben:
R¹, R² Wasserstoff, Mercapto, Nitro, Halogen, Cyano, Rhodano, C₁-C₆-Alkyl, C₁-C₆-Halogenalkyl, C₁-C₆-Alkoxy, C₂-C₆-Alkenyl, C₂-C₆-Alkinyl, -OR³, -OCOR³, -OSO₂R³, -S(O)ₙR³, -SO₂OR³, -SO₂N(R³)₂, -NR³SO₂R³ oder -NR³COR³;
R³ Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/ oder eine bis drei der folgenden Gruppen tragen können:
   Hydroxy, Mercapto, Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
n 0,1 oder 2;
X¹ eine geradkettige oder verzweigte C₁-C₆-Alkylen-, eine C₂-C₆-Alkenylen- oder eine C₂-C₆-Alkinylenkette,die durch ein Heteroatom ausgewählt aus der Gruppe:
   Sauerstoff oder Schwefel
   unterbrochen ist und wobei die genannten Alkylen-,
      Alkenylen- oder Alkinylenreste partiell halogeniert sein können und/oder eine bis drei der folgenden Gruppen tragen können:

      -OR⁴, -OCOR⁴, -OCONHR⁴ oder -OSO₂R⁴;
R⁴ Wasserstoff, C₁-C₆-Alkyl, C₂-C₆-Alkenyl, C₂-C₆-Alkinyl, Phenyl, Phenyl-C₁-C₆-alkyl, wobei die genannten Alkyl-, Alkenyl oder Alkinylreste partiell oder vollständig halogeniert sein können und/oder durch einen oder mehrere der folgenden Reste substituiert sein können:
   Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
Het eine drei- bis sechsgliedrige, teilweise oder vollständig gesättigte, heterocyclische Gruppe oder eine drei- bis sechsgliedrige heteroaromatische Gruppe mit bis zu drei Heteroatomen ausgewählt aus der Gruppe:
   Stickstoff, Sauerstoff oder Schwefel,
   wobei die gennante heterocyclische oder heteroaromatische Gruppe partiell oder vollständig halogeniert sein kann und/oder durch R⁵ substituiert sein kann;
R⁵ Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
   Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy;
R¹² Hydroxy oder ein hydrolysierbarer Rest.
   Beispiele für hydrolysierbare Reste sind Alkoxy-, Phenoxy-, Alkylthio-, Phenylthioreste, die substituiert sein können, Halogenide, Hetarylreste, die über Stickstoff gebunden sind, Amino-, Iminoreste, die substituiert sein können, etc.
   Bevorzugt sind Benzoesäurehalogenide IIIa mit L¹ = Halogen (=̂ III mit R¹² = Halogen), 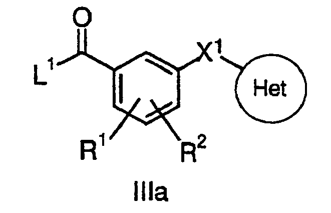 wobei die Variablen R¹, R², X und Het die unter Formel III genannte Bedeutung haben und
L¹ Halogen, insbesondere Chlor oder Brom, bedeuten.
   Ebenso bevorzugt sind Benzoesäuren der Formel IIIb (=̂ III mit R¹²= Hydroxy), 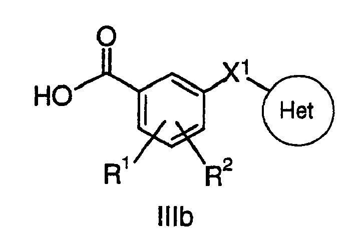 wobei die Variablen R¹, R², X¹ und Het die unter Formel III genannte Bedeutung haben.
   Ebenso bevorzugt sind Benzoesäureester der Formel IIIc (=̂ III mit R¹² = C₁-C₆-Alkoxy), 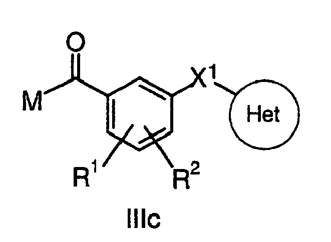 wobei die Variablen R¹, R², X¹ und Het die unter Formel III genannte Bedeutung haben und
M C₁-C₆-Alkoxy
   bedeutet.

Die besonders bevorzugten Ausführungsfernen der Benzoesäuren der Formel III in Bezug auf die Variablen R¹, R², X¹ und Het entsprechen denen der 2-Benzoylcyclohexan-1,3-dione der Formel I.

Die Verbindungen der Formel IIIa (mit L¹ = Halogen) können in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel IIIb mit Halogenierungsreagentien wie Thionylchlorid, Thionylbromid, Phosgen, Diphosgen, Triphosgen, Oxalylchlorid, Oxalylbromid dargestellt werden.

Die Benzoesäuren der Formel IIIb können u. a. durch Verseifung der Benzoesäureester der Formel IIIc (mit M = C₁-C₆-Alkoxy) erhalten werden.

Die erfindungsgemäßen Benzoesäureester der Formel IIIc sind nach verschiedenen literaturbekannten Methoden (z. B. a. G. Dittus in Houben-Weyl, Methoden der Organischen Chemie, Band VI/3, Sauerstoff-Verbindungen I, 4. Aufl., S. 493 ff., Georg Thieme Verlag, 1965; b. T. L. Gilchrist, Heterocyclenchemie, 2. Aufl., Verlag Chemie, 1995) darstellbar, wie in den nachfolgenden Beispielen illustriert.

Verfahren B:
Die Substitution der Benzoesäureester Va mit geeigneten Nucleophilen VI liefert die erfindungsgemäßen Benzoesäureester IIIc, 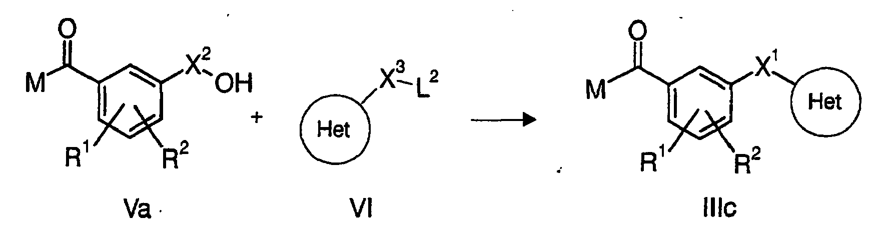 wobei M, R¹ und R² die oben genannte Bedeutung haben, L² eine geeignete, nucleophil austauschbare Abgangsgruppe, wie Halogen, z.B. Brom, Chlor, Hetaryl, z.B. Imidazolyl, Pyridyl, Carboxylat, z.B. Acetat, Trifluoracetat, Sulfonat, z.B. Mesylat, Triflat etc. darstellt,
X² eine geradkettige oder verzweigte Alkylen-, eine Alkenylen-oder eine Alkinylenkette mit mindestens einem und höchstens fünf Kohlenstoffatomen darstellt,
   wobei die genannten Alkylen-, Alkenylen- oder Alkinylenreste partiell halogeniert sein können und/oder eine bis drei der folgenden Gruppen tragen können:

   -OR⁴, -OCOR⁴, -OCONHR⁴ oder -OSO₂R⁴ und
X³ eine geradkettige oder verzweigte Alkylen-, eine Alkenylen-oder eine Alkinylenkette mit höchstens fünf Kohlenstoffatomen darstellt,
   wobei die genannten Alkylen-, Alkenylen- oder Alkinylenreste partiell halogeniert sein können und/oder eine bis drei der folgenden Gruppen tragen können:

   -OR⁴, -OCOR⁴, -OCONHR⁴ oder -OSO₂R⁴.
wobei X²OX³ die Variable X¹ ausbilden.

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf Va, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin, Pyridin und Kaliumcarbonat.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-*tert*-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise erfolgen.
Verfahren C:
Die Substitution der geeignet substituierten Heterocyclen VII mit den Benzoesäureestern Vb liefert die erfindungsgemäßen Benzoesäureester IIIc, 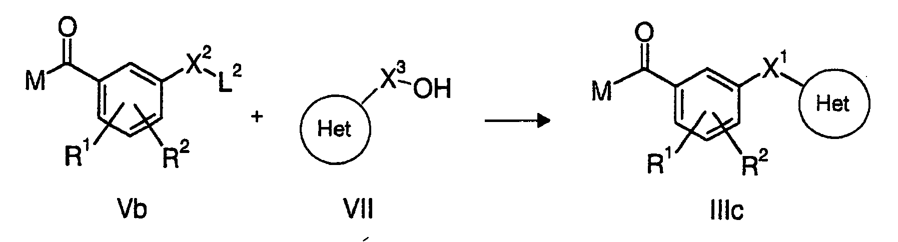 wobei M, R¹ und R² die oben genannte Bedeutung haben, L² eine geeignete, nucleophil austauschbare Abgangsgruppe, wie Halogen, z.B. Brom, Chlor, Hetaryl, z.B. Imidazolyl, Pyridyl, Carboxylat,
   z.B. Acetat, Trifluoracetat, Sulfonat, z.B. Mesylat, Triflat etc. darstellt,
X² eine geradkettige oder verzweigte Alkylen-, eine Alkenylen-oder eine Alkinylenkette mit mindestens einem und höchstens fünf Kohlenstoffatomen darstellt,
   wobei die genannten Alkylen-, Alkenylen- oder Alkinylenreste partiell halogeniert sein können und/oder eine bis drei der folgenden Gruppen tragen können:

   -OR⁴, -OCOR⁴, -OCONHR⁴ oder -OSO₂R⁴ und
X³ eine geradkettige oder verzweigte Alkylen-, eine Alkenylen-oder eine Alkinylenkette mit höchstens fünf Kohlenstoffatomen darstellt,
   wobei die genannten Alkylen-, Alkenylen- oder Alkinylenreste partiell halogeniert sein können und/oder eine bis drei der folgenden Gruppen tragen können:

   -OR⁴, -OCOR⁴, -OCONHR⁴ oder -OSO₂R⁴,

   wobei X²OX³ die Variable X¹ ausbilden.

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf VII, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin, Pyridin und Kaliumcarbonat.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-*tert*-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise erfolgen.

Hervorzuheben sind die erfindungsgemäßen Verbindungen der Formel I, wobei die Gruppe X¹ entweder für eine C₁-C₂-Alkylen- oder eine C₂-Alkenylenkette, die ein weiteres Sauerstoff- oder Schwefelatom enthält, steht und

Het eine drei- bis sechsgliedrige, teilweise oder vollständig gesättigte, heterocyclische Gruppe oder eine drei- bis sechsgliedrige heteroaromatische Gruppe mit bis zu drei Heteroatomen ausgewählt aus der Gruppe:
Stickstoff, Sauerstoff oder Schwefel,
   wobei die gennante heterocyclische oder heteroaromatische Gruppe partiell oder vollständig halogeniert sein kann und/oder durch R⁵ substituiert sein kann,
darstellt.

Darüber hinaus sind die erfindungsgemäßen Verbindungen der Formel I hervorzuheben, wobei die Gruppe Het für eine fünf- oder sechsgliedrige, teilweise oder vollständig gesättigte heterocyclische oder eine fünf- oder sechsgliedrige heteroaromatische Gruppe mit bis zu drei Heteroatomen ausgewählt aus der Gruppe Stickstoff, Sauerstoff oder Schwefel steht, wobei die gennante heterocyclische oder heteroaromatische Gruppe partiell oder vollständig halogeniert und/oder durch R⁵ substituiert sein kann;
R⁵ Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können: Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy.

Die für die Substituenten R¹ - R¹² oder als Reste an Phenyl-, Hetaryl- und Heterocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Alkyliminooxy-, Alkoxyamino-, Alkylsulfonyl-, Halogenalkylsulfonyl, Alkylcarbonyl-, Halogenalkylcarbonyl, Alkoxycarbonyl-, Alkoxyalkoxycarbonyl-, Alkenyl-, Cycloalkenyl-, Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl, sowie die Alkylteile von C₁-C₄-Alkylcarbonyl: Methyl, Ethyl, n-Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkoxy-C₁-C₆-alkyl und C₁-C₆-Alkylcarbonyl: C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl, sowie die Halogenalkylteile von C₁-C₆-Halogenalkylcarbonyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy, sowie die Alkoxyteile von C₁-C₄-Alkoxyamino, C₁-C₄-Alkoxy-C₂-C₆-alkoxycarbonyl und C₁-C₄-Alkoxycarbonyl: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile von C₁-C₆-Alkoxy-C₁-C₆-alkyl, C₁-C₆-Alkoxy-C₂-C₆-alkyl, C₁-C₄-Alkoxy-C₂-C₆-alkoxycarbonyl und C₁-C₆-Alkoxycarbonyl: C₁-C₄-Alkoxy wie voranstehend genannt, sowie Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z. B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₄-Alkylsulfonyl (C₁-C₄-Alkyl-S(=O)₂-): Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl: C₁-C₄-Alkylsulfonyl wie voranstehend genannt, sowie Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₆-Halogenalkylsulfonyl: einen C₁-C₆-Alkylsulfonylrest wie voranstehend genannt, der partiell oder vollständig durch Fluor, Chlor, Brom und/ oder Iod substituiert ist, also Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkyliminooxy: Methyliminooxy, Ethyliminooxy, 1-Propyliminooxy, 2-Propyliminooxy, 1-Butyliminooxy und 2-Butyliminooxy;
- C₃-C₆-Alkenyl: Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-eh-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-l-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- C₂-C₆-Alkinyl: C₃-C₆-Alkinyl, wie voranstehend genannt, sowie Ethinyl:
- C₃-C₆-Cycloalkyl: Cyclopropyl, Cyclobutyl, Cyclopentyl und Cyclohexyl;
- C₄-C₆-Cycloalkenyl: Cyclobuten-1-yl, Cyclobuten-3-yl, Cyclopenten-1-yl, Cyclopenten-3-yl, Cyclopenten-4-yl, Cyclohexen-1-yl, Cyclohexen-3-yl und Cyclohexen-4-yl;
- Heterocyclyl, sowie die Heteroclylreste in Heterocyclyloxy: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazal-4-yl, 2,5-Dihydroisoxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl,
   3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl,
- Hetaryl, sowie die Hetarylreste in Hetaryloxy: aromatische mono- oder polycyclische Reste, welche neben Kohlenstoffringgliedern zusätzlich ein bis vier Stickstoffatome oder ein bis drei Stickstoffatome und ein Sauerstoff-oder ein Schwefelatom oder ein Sauerstoff- und ein Schwefelatom enthalten können, z. B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate.

Alle Phenyl-, Hetaryl- und Heterocyclylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
R¹ Nitro, Halogen, Cyano, Rhodano, C₁-C₆-Alkyl, C₁-C₆-Halogen-
   alkyl, C₁-C₆-Alkoxy-C₁-C₆-alkyl, C₂-C₆-Alkenyl, C₂-C₆-Alkinyl, -OR³ oder -S(O)ₙR³;
   besonders bevorzugt Nitro, Halogen wie z.B. Fluor, Chlor oder Brom, C₁-C₆-Halogenalkyl, -OR³ oder -SO₂R³;
R² Wasserstoff, Nitro, Halogen, Cyano, Rhodano, C₁-C₆-Alkyl,
   C₁-C₆-Halogenalkyl, C₁-C₆-Alkoxy-C₁-C₆-alkyl, C₂-C₆-Alkenyl, C₂-C₆-Alkinyl, -OR³ oder -S(O)n_{R}³;
   besonders bevorzugt Wasserstoff, Nitro, Halogen wie z.B. Fluor, Chlor oder Brom, C₁-C₆-Alkyl, C₁-C₆-Halogenalkyl, -OR³ oder -SO₂R³;
R³ Wasserstoff, C₁-C₆-Alkyl, C₁-C₆-Halogenalkyl, C₂-C₆-Alkenyl,
   C₂-C₆-Alkinyl, Phenyl oder Phenyl-C₁-C₆-alkyl;
   besonders bevorzugt Wasserstoff, C₁-C₄-Alkyl, C₁-C₄-Halogenalkyl, C₂-C₃-Alkenyl, C₂-C₃-Alkinyl oder Phenyl; wobei die genannten Alkylreste partiell oder vollständig halogeniert sein können und/oder eine bis drei der folgenden Gruppen tragen können:
   Hydroxy, Mercapto, Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
   ebenso bevorzugt Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/oder eine bis drei der folgenden Gruppen tragen können:
   Hydroxy, Mercapto, Amino, Cyano, R^{3'}, -OR^{3'}, -SR^{3'}, -N(R^{3'})_{2,} =NOR^{3'}, -OCOR^{3'}, -SCOR^{3'}, NR^{3'}COR^{3'}, CO₂R^{3'}, -COSR^{3'}, -CON(R^{3'})₂, C₁-C₄-Alkyliminoxy, C₂-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
      (mit R^{3'} Wasserstoff, C₁-C₆-Alkyl, C₁-C₆-Halogenalkyl, C₂-C₆-Alkenyl, C₂-C₆-Alkinyl, Phenyl oder Phenyl-C₁-C₆-alkyl);
n 0,1 oder 2, besonders bevorzugt 0 oder 2;
X¹ eine geradkettige oder verzweigte C₁-C₄-Alkylen-, eine C₂-C₄-Alkenylen- oder eine C₂-C₄-Alkinylenkette, besonders bevorzugt eine Ethylen-, Propylen-, Propenylen- oder Propinylenkette, die durch ein Heteroatom ausgewählt aus der Gruppe
   Sauerstoff oder Schwefel, bevorzugt Sauerstoff
   unterbrochen ist, wobei die genannten Alkylen-, Alkenylen-oder Alkinylenreste partiell halogeniert sein können und/oder eine bis drei der folgenden Gruppen tragen können:

   -OR⁴, -OCOR⁴, -OCONHR⁴ oder -OSO₂R⁴;
R⁴ Wasserstoff, C₁-C₆-Alkyl oder C₁-C₆-Halogenalkyl; besonders bevorzugt Wasserstoff, Methyl, Ethyl oder Trifluormethyl;
R⁵ Wasserstoff, Hydroxy, Mercapto, Amino, Cyano, Nitro, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
   Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy.
R⁶, R⁷, R⁹, R¹¹ Wasserstoff oder C₁-C₄-Alkyl; besonders bevorzugt Wasserstoff, Methyl oder Ethyl;
R⁸ Wasserstoff, C₁-C₄-Alkyl, C₃-C₄-Cycloalkyl, wobei die beiden letztgenannten Gruppen gegebenenfalls einen bis drei der folgenden Substituenten tragen können: Halogen, C₁-C₄-Alkoxy oder C₁-C₄-Alkylthio; Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,3-Oxathiolan-2-yl, 1,3-Oxathian-2-yl, 1,3-Dithian-2-yl oder 1,3-Dithiolan-2-yl, wobei die sechs letztgenannten Gruppen jeweils einen bis drei C₁-C₄-Alkylreste tragen können; besonders bevorzugt Wasserstoff, Methyl, Ethyl, Cyclopropyl, Di(methoxy)methyl, Di(ethoxy)methyl, 2-Ethylthiopropyl, Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 5,5-Dimethyl-1-3-dioxan-2-yl, 1,3-Oxathiolan-2-yl, 1,3-Oxathian-2-yl, 1,3-Dithiolan-2-yl, 5,5-Dimethyl-1,3-dithian-2-yl oder 1-Methylthiocyclopropyl;
R¹⁰ Wasserstoff, C₁-C₄-Alkyl oder C₁-C₄-Alkoxycarbonyl; besonders bevorzugt Wasserstoff, Methyl oder Methoxycarbonyl.
Ebenso kann vorteilhaft in Betracht kommen, daß R⁸ und R¹¹ eine π-Bindung ausbilden, so daß ein Doppelbindungssystem entsteht.

Die CR⁸R⁹-Einheit kann auch vorteilhaft durch C=O ersetzt werden.

Insbesondere bevorzugt sind die Verbindungen der Formel Ia, wobei R¹ in Position 2 und R² in Position 4 des Phenylringes gebunden sind. 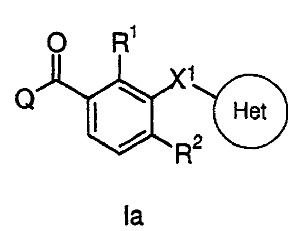 Außerordentlich bevorzugt sind die Verbindungen der Formel Ia, in der die Substituenten R¹, R² und Q die oben genannte Bedeutung haben, X¹ für eine C₁-C₂-Alkylen- oder eine C₂-Alkinylenkette, die ein weiteres Sauerstoffatom enthält, steht und

Het eine drei- bis sechsgliedrige, bevorzugt eine fünf- oder sechsgliedrige, teilweise oder vollständig gesättigte, heterocyclische Gruppe oder eine drei- bis sechsgliedrige, bevorzugt fünf- oder sechsgliedrige heteroaromatische Gruppe mit bis zu drei Heteroatomen, besonders bevorzugt mit einem oder zwei Heteroatomen ausgewählt aus folgenden drei Gruppen:
Stickstoff,
Sauerstoff in Kombination mit mindestens einem Stickstoff oder
Schwefel in Kombination mit mindestens einem Stickstoff,
besonders bevorzugt aus folgenden beiden Gruppen:
   Stickstoff oder
   Sauerstoff in Kombination mit mindestens einem Stickstoff,
   wobei die gennante heterocyclische oder heteroaromatische Gruppe partiell oder vollständig halogeniert sein kann und/ oder durch R⁵ substituiert sein kann;
bedeutet.

Darüber hinaus sind die erfindungsgemäßen Verbindungen der Formel Ia außerordentlich bevorzugt, in der die Substituenten R¹, R² und X¹ die oben genannte Bedeutung haben und Het für eine fünf-oder sechsgliedrige, teilweise oder vollständig gesättigte, heterocyclische Gruppe oder eine fünf- oder sechsgliedrige heteroaromatische Gruppe mit bis zu drei Heteroatomen, besonders bevorzugt mit einem oder zwei Heteroatomen ausgewählt aus folgenden drei Gruppen:
Stickstoff,
   Sauerstoff in Kombination mit mindestens einem Stickstoff oder
   Schwefel in Kombination mit mindestens einem Stickstoff,
besonders bevorzugt aus folgenden beiden Gruppen:
   Stickstoff oder
      Sauerstoff in Kombination mit mindestens einem Stickstoff,
      wobei die gennante heterocyclische oder heteroaromatische Gruppe partiell oder vollständig halogeniert sein kann und/oder durch R⁵ substituiert sein kann;
steht.

Insbesondere bevorzugt sind die Verbindungen Ib der Tabellen 1 bis 36.

**Tabelle A**

| Nr. | X¹ * | Het |
|---|---|---|
| 1 | OCH₂ | Oxiranyl |
| 2 | OCH₂ | 3-Methyl-2-oxiranyl |
| 3 | OCH₂ | 2-Oxetanyl |
| 4 | OCH₂ | 3-Aydroxy-3-methyl-2-oxetanyl |
| 5 | OCH₂ | 3-Hydroxy-3-ethyl-2-oxetanyl |
| 6 | OCH₂ | 3-Hydroxy-3-propyl-2-oxetanyl |
| 7 | OCH₂ | 3-Hydroxy-3-butyl-2-oxetanyl |
| 8 | OCH₂ | 3-Methoxy-3-methyl-2-oxetanyl |
| 9 | OCH₂ | 3-Methoxy-3-ethyl-2-oxetanyl |
| 10 | OCH₂ | 3-Methoxy-3-propyl-2-oxetanyl |
| 11 | OCH₂ | 3-Methoxy-3-butyl-2-oxetanyl |
| 12 | OCH₂ | 3-Trimethylsilyl-oxy-3-methyl-2-oxetanyl |
| 13 | OCH₂ | 3-Trimethylsilyl-oxy-3-ethyl-2-oxetanyl |
| 14 | OCH₂ | 3-Trimethylsilyl-oxy-3-propyl-2-oxetanyl |
| 15 | OCH₂ | 3-Trimethylsilyl-oxy-3-butyl-2-oxetanyl |
| 16 | OCH₂ | 3-Oxetanyl |
| 17 | OCH₂ | 2-Furyl |
| 18 | OCH₂ | 4,5-Dihydro-2-furyl |
| 19 | OCH₂ | 2,3-Dihydro-2-furyl |
| 20 | OCH₂ | 3-Furyl |
| 21 | OCH₂ | 4,5-Dihydro-3-furyl |
| 22 | OCH₂ | 2,3-Dihydro-3-furyl |
| 23 | OCH₂ | 2-Thienyl |
| 24 | OCH₂ | 4,5-Dihydro-2-thienyl |
| 25 | OCH₂ | 2,3-Dihydro-2-thienyl |
| 26 | OCH₂ | 5-Chlor-2-thienyl |
| 27 | OCH₂ | 5-Methyl-2-thienyl |
| 28 | OCH₂ | 3-Thienyl |
| 29 | OCH₂ | 4,5-Dihydro-3-thienyl |
| 30 | OCH₂ | 2,3-Dihydro-3-thienyl |
| 31 | OCH₂ | 2-Pyrrolyl |
| 32 | OCH₂ | 2,5-Dihydro-2-pyrrolyl |
| 33 | OCH₂ | 3-Pyrrolyl |
| 34 | OCH₂ | 2,5-Dihydro-3-pyrrolyl |
| 35 | OCH₂ | 3-Isoxazolyl |
| 36 | OCH₂ | 4-Methyl-3-isoxazolyl |
| 37 | OCH₂ | 5-Methyl-3-isoxazolyl |
| 38 | OCH₂ | 4,5-Dimethyl-3-isoxazolyl |
| 39 | OCH₂ | 4,5-Dihydro-3-isoxazolyl |
| 40 | OCH₂ | 4-Methyl-4,5-dihydro-3-isoxazolyl |
| 41 | OCH₂ | 5-Methyl-4,5-dihydro-3-isoxazolyl |
| 42 | OCH₂ | 4,5-Dimethyl-4,5-di-hydro-3-isoxazolyl |
| 43 | OCH₂ | 4-Isoxazolyl |
| 44 | OCH₂ | 3-Methyl-4-isoxazolyl |
| 45 | OCH₂ | 5-Methyl-4-isoxazolyl |
| 46 | OCH₂ | 5-Cyclopropyl-4-isoxazolyl |
| 47 | OCH₂ | 5-Phenyl-4-isoxazolyl |
| 48 | OCH₂ | 3,5-Dimethyl-4-isoxazolyl |
| 49 | OCH₂ | 4,5-Dihydro-4-isoxazolyl |
| 50 | OCH₂ | 3-Methyl-4,5-dihydro-4-isoxazolyl |
| 51 | OCH₂ | 5-Methyl-4,5-dihydro-4-isoxazolyl |
| 52 | OCH₂ | 3,5-Dimethyl-4,5-di-hydro-4-isoxazolyl |
| 53 | OCH₂ | 5-Isoxazolyl |
| 54 | OCH₂ | 3-Methyl-5-isoxazolyl |
| 55 | OCH₂ | 4-Methyl-5-isoxazolyl |
| 56 | OCH₂ | 3,4-Dimethyl-5-isoxazolyl |
| 57 | OCH₂ | 4,5-Dihydro-5-isoxazolyl |
| 58 | OCH₂ | 3-Methyl-4,5-dihydro-5-isoxazolyl |
| 59 | OCH₂ | 4-Methyl-4,5-dihydro-5-isoxazolyl |
| 60 | OCH₂ | 3,4-Dimethyl-4,5-di-hydro-5-isoxazolyl |
| 61 | OCH₂ | 3-Isothiazolyl |
| 62 | OCH₂ | 4-Methyl-3-isothiazolyl |
| 63 | OCH₂ | 5-Methyl-3-isothiazolyl |
| 64 | OCH₂ | 4-Isothiazolyl |
| 65 | OCH₂ | 3-Methyl-4-isothiazolyl |
| 66 | OCH₂ | 5-Methyl-4-isothiazolyl |
| 67 | OCH₂ | 5-Isothiazolyl |
| 68 | OCH₂ | 3-Methyl-5-isothiazolyl |
| 69 | OCH₂ | 4-Methyl-5-isothiazolyl |
| 70 | OCH₂ | 2-Oxazolyl |
| 71 | OCH₂ | 4-Oxazolyl |
| 72 | OCH₂ | 5-Oxazolyl |
| 73 | OCH₂ | 2-Thiazolyl |
| 74 | OCH₂ | 4-Thiazolyl |
| 75 | OCH₂ | 5-Thiazolyl |
| 76 | OCH₂ | 3-Pyrazolyl |
| 77 | OCH₂ | 4-Pyrazolyl |
| 78 | OCH₂ | 1-Methyl-3-pyrazolyl |
| 79 | OCH₂ | 1-Methyl-4-pyrazolyl |
| 80 | OCH₂ | 1-Methyl-5-pyrazolyl |
| 81 | OCH₂ | 2-Imidazolyl |
| 82 | OCH₂ | 1-Methyl-2-imidazolyl |
| 83 | OCH₂ | 5-Methyl-[1,3,4]-2-oxadiazolyl |
| 84 | OCH₂ | 5-Methyl-[1,2,4]-3-oxadiazolyl |
| 85 | OCH₂ | 5-Methyl-[1,3,4]-2-thiadiazolyl |
| 86 | OCH₂ | 5-Methyl-[1,2,4]-3-thiadiazolyl |
| 87 | OCH₂ | [1,2,4]-3-triazolyl |
| 88 | OCH₂ | [1,2,3]-4-triazolyl |
| 89 | OCH₂ | 2-Pyridyl |
| 90 | OCH₂ | 6-Chlor-2-pyridyl |
| 91 | OCH₂ | 6-Methoxy-2-pyridyl |
| 92 | OCH₂ | 6-Trifluormethyl-2-pyridyl |
| 93 | OCH₂ | 3-Pyridyl |
| 94 | OCH₂ | 2-Chlor-3-pyridyl |
| 95 | OCH₂ | 2-Methoxy-3-pyridyl |
| 96 | OCH₂ | 4-Pyridyl |
| 97 | OCH₂ | 2-Chlor-4-pyridyl |
| 98 | OCH₂ | 2-Methoxy-4-pyridyl |
| 99 | OCH₂ | 2-Ethoxy-4-pyridyl |
| 100 | OCH₂ | 2-Methylthio-4-pyridyl |
| 101 | OCH₂ | 2-Trifluormethyl-5-pyridyl |
| 102 | OCH₂ | 2-Pyrimidinyl |
| 103 | OCH₂ | 3-Pyrimidinyl |
| 104 | OCH₂ | 4-Pyrimidinyl |
| 105 | OCH₂ | 2-Pyrazinyl |
| 106 | OCH₂ | 3-Pyridazinyl |
| 107 | OCH₂ | 4-Pyridazinyl |
| 108 | OCH₂ | 2-(2H-1,3-oxazinyl) |
| 109 | OCH₂ | 2-(6*H*-1,3-oxazinyl) |
| 110 | OCH₂ | 4-(6*H*-1,3-oxazinyl) |
| 111 | OCH₂ | 6-(6*H*-1,3-oxazinyl) |
| 112 | OCH₂ | [1,3.5]-2-Triazinyl |
| 113 | OCH₂ | [1,2,4]-3-Triazinyl |
| 114 | OCH₂ | [1,2,4]-5-Triazinyl |
| 115 | OCH₂ | [1,2,4]-6-Triazinyl |
| 116 | CH₂O | Oxiranyl |
| 117 | CH₂O | 3-Methyl-2-oxiranyl |
| 118 | CH₂O | 2-Oxetanyl |
| 119 | CH₂O | 3-Hydroxy-3-methyl-2-oxetanyl |
| 120 | CH₂O | 3-Hydroxy-3-ethyl-2-oxetanyl |
| 121 | CH₂O | 3-Hydroxy-3-propyl-2-oxetanyl |
| 122 | CH₂O | 3-Hydroxy-3-butyl-2-oxetanyl |
| 123 | CH₂O | 3-Methoxy-3-methyl-2-oxetanyl |
| 124 | CH₂O | 3-Methoxy-3-ethyl-2-oxetanyl |
| 125 | CH₂O | 3-Methoxy-3-propyl-2-oxetanyl |
| 126 | CH₂O | 3-Methoxy-3-butyl-2-oxetanyl |
| 127 | CH₂O | 3-Trimethylsilyl-oxy-3-methyl-2-oxetanyl |
| 128 | CH₂O | 3-Trimethylsilyl-oxy-3-ethyl-2-oxetanyl |
| 129 | CH₂O | 3-Trimethylsilyl-oxy-3-propyl-2-oxetanyl |
| 130 | CH₂O | 3-Trimethylsilyl-oxy-3-butyl-2-oxetanyl |
| 131 | CH₂O | 3-Oxetanyl |
| 132 | CH₂O | 2-Furyl |
| 133 | CH₂O | 4,5-Dihydro-2-furyl |
| 134 | CH₂O | 2,3-Dihydro-2-furyl |
| 135 | CH₂O | 3-Furyl |
| 136 | CH₂O | 4,5-Dihydro-3-furyl |
| 137 | CH₂O | 2,3-Dihydro-3-furyl |
| 138 | CH₂O | 2-Thienyl |
| 139 | CH₂O | 4,5-Dihydro-2-thienyl |
| 140 | CH₂O | 2,3-Dihydro-2-thienyl |
| 141 | CH₂O | 5-Chlor-2-thienyl |
| 142 | CH₂O | 5-Methyl-2-thienyl |
| 143 | CH₂O | 3-Thienyl |
| 144 | CH₂O | 4,5-Dihydro-3-thienyl |
| 145 | CH₂O | 2,3-Dihydro-3-thienyl |
| 146 | CH₂O | 2-Pyrrolyl |
| 147 | CH₂O | 2,5-Dihydro-2-pyrrolyl |
| 148 | CH₂O | 3-Pyrrolyl |
| 149 | CH₂O | 2,5-Dihydro-3-pyrrolyl |
| 150 | CH₂O | 3-Isoxazolyl |
| 151 | CH₂O | 4-Methyl-3-isoxazolyl |
| 152 | CH₂O | 5-Methyl-3-isoxazolyl |
| 153 | CH₂O | 4,5-Dimethyl-3-isoxazolyl |
| 154 | CH₂O | 4,5-Dihydro-3-isoxazolyl |
| 155 | CH₂O | 4-Methyl-4,5-dihydro-3-isoxazolyl |
| 156 | CH₂O | 5-Methyl-4,5-dihydro-3-isoxazolyl |
| 157 | CH₂O | 4,5-Dimethyl-4,5-di-hydro-3-isoxazolyl |
| 158 | CH₂O | 4-Isoxazolyl |
| 159 | CH₂O | 3-Methyl-4-isoxazolyl |
| 160 | CH₂O | 5-Methyl-4-isoxazolyl |
| 161 | CH₂O | 5-Cyclopropyl-4-isoxazolyl |
| 162 | CH₂O | 5-Phenyl-4-isoxazolyl |
| 163 | CH₂O | 3,5-Dimethyl-4-isoxazolyl |
| 164 | CH₂O | 4,5-Dihydro-4-isoxazolyl |
| 165 | CH₂O | 3-Methyl-4,5-dihydro-4-isoxazolyl |
| 166 | CH₂O | 5-Methyl-4,5-dihydro-4-isoxazolyl |
| 167 | CH₂O | 3,5-Dimethyl-4,5-di-hydro-4-isoxazolyl |
| 168 | CH₂O | 5-Isoxazolyl |
| 169 | CH₂O | 3-Methyl-5-isoxazolyl |
| 170 | CH₂O | 4-Methyl-5-isoxazolyl |
| 171 | CH₂O | 3,4-Dimethyl-5-isoxazolyl |
| 172 | CH₂O | 4,5-Dihydro-5-isoxazolyl |
| 173 | CH₂O | 3-Methyl-4,5-dihydro-5-isoxazolyl |
| 174 | CH₂O | 4-Methyl-4,5-dihydro-5-isoxazolyl |
| 175 | CH₂O | 3,4-Dimethyl-4,5-di-hydro-5-isoxazolyl |
| 176 | CH₂O | 3-Isothiazolyl |
| 177 | CH₂O | 4-Methyl-3-isothiazolyl |
| 178 | CH₂O | 5-Methyl-3-isothiazolyl |
| 179 | CH₂O | 4-Isothiazolyl |
| 180 | CH₂O | 3-Methyl-4-isothiazolyl |
| 181 | CH₂O | 5-Methyl-4-isothiazolyl |
| 182 | CH₂O | 5-Isothiazolyl |
| 183 | CH₂O | 3-Methyl-5-isothiazolyl |
| 184 | CH₂O | 4-Methyl-5-isothiazolyl |
| 185 | CH₂O | 2-Oxazolyl |
| 186 | CH₂O | 4-Oxazolyl |
| 187 | CH₂O | 5-Oxazolyl |
| 188 | CH₂O | 2-Thiazolyl |
| 189 | CH₂O | 4-Thiazolyl |
| 190 | CH₂O | 5-Thiazolyl |
| 191 | CH₂O | 3-Pyrazolyl |
| 192 | CH₂O | 4-Pyrazolyl |
| 193 | CH₂O | 1-Methyl-3-pyrazolyl |
| 194 | CH₂O | 1-Methyl-4-pyrazolyl |
| 195 | CH₂O | 1-Methyl-5-pyrazolyl |
| 196 | CH₂O | 2-Imidazolyl |
| 197 | CH₂O | 1-Methyl-2-imidazolyl |
| 198 | CH₂O | 5-Methyl-[1,3,4]-2-oxadiazolyl |
| 199 | CH₂O | 5-Methyl-[1,2,4]-3-oxadiazolyl |
| 200 | CH₂O | 5-Methyl-[1,3,4]-2-thiadiazolyl |
| 201 | CH₂O | 5-Methyl-[1,2,4]-3-thiadiazolyl |
| 202 | CH₂O | [1,2,4]-3-triazolyl |
| 203 | CH₂O | [1,2,3]-4-triazolyl |
| 204 | CH₂O | 2-Pyridyl |
| 205 | CH₂O | 6-Chlor-2-pyridyl |
| 206 | CH₂O | 6-Methoxy-2-pyridyl |
| 207 | CH₂O | 6-Trifluormethyl-2-pyridyl |
| 208 | CH₂O | 3-Pyridyl |
| 209 | CH₂O | 2-Chlor-3-pyridyl |
| 210 | CH₂O | 2-Methoxy-3-pyridyl |
| 211 | CH₂O | 4-Pyridyl |
| 212 | CH₂O | 2-Chlor-4-pyridyl |
| 213 | CH₂O | 2-Methoxy-4-pyridyl |
| 214 | CH₂O | 2-Ethoxy-4-pyridyl |
| 215 | CH₂O | 2-Methylthio-4-pyridyl |
| 216 | CH₂O | 2-Trifluormethyl-5-pyridyl |
| 217 | CH₂O | 2-Pyrimidinyl |
| 218 | CH₂O | 3-Pyrimidinyl |
| 219 | CH₂O | 4-Pyrimidinyl |
| 220 | CH₂O | 2-Pyrazinyl |
| 221 | CH₂O | 3-Pyridazinyl |
| 222 | CH₂O | 4-Pyridazinyl |
| 223 | CH₂O | 2-(2*H*-1,3-oxazinyl) |
| 224 | CH₂O | 2-(6*H*-1,3-oxazinyl) |
| 225 | CH₂O | 4-(6*H*-1,3-oxazinyl) |
| 226 | CH₂O | 6-(6*H*-1,3-oxazinyl) |
| 227 | CH₂O | [1,3,5]-2-Triazinyl |
| 228 | CH₂O | [1,2,4]-3-Triazinyl |
| 229 | CH₂O | [1,2,4]-5-Triazinyl |
| 230 | CH₂O | [1,2,4]-6-Triazinyl |
| 231 | OCH₂CH₂ | Oxiranyl |
| 232 | OCH₂CH₂ | 3-Methyl-2-oxiranyl |
| 233 | OCH₂CH₂ | 2-Oxetanyl |
| 234 | OCH₂CH₂ | 3-Hydroxy-3-methyl-2-oxetanyl |
| 235 | OCH₂CH₂ | 3-Hydroxy-3-ethyl-2-oxetanyl |
| 236 | OCH₂CH₂ | 3-Hydroxy-3-propyl-2-oxetanyl |
| 237 | OCH₂CH₂ | 3-Hydroxy-3-butyl-2-oxetanyl |
| 238 | OCH₂CH₂ | 3-Methoxy-3-methyl-2-oxetanyl |
| 239 | OCH₂CH₂ | 3-Methoxy-3-ethyl-2-oxetanyl |
| 240 | OCH₂CH₂ | 3-Methoxy-3-propyl-2-oxetanyl |
| 241 | OCH₂CH₂ | 3-Methoxy-3-butyl-2-oxetanyl |
| 242 | OCH₂CH₂ | 3-Trimethylsilyl-oxy-3-methyl-2-oxetanyl |
| 243 | OCH₂CH₂ | 3-Trimethylsilyl-oxy-3-ethyl-2-oxetanyl |
| 244 | OCH₂CH₂ | 3-Trimethylsilyl-oxy-3-propyl-2-oxetanyl |
| 245 | OCH₂CH₂ | 3-Trimethylsilyl-oxy-3-butyl-2-oxetanyl |
| 246 | OCH₂CH₂ | 3-Oxetanyl |
| 247 | OCH₂CH₂ | 2-Furyl |
| 248 | OCH₂CH₂ | 4,5-Dihydro-2-furyl |
| 249 | OCH₂CH₂ | 2,3-Dihydro-2-furyl |
| 250 | OCH₂CH₂ | 3-Furyl |
| 251 | OCH₂CH₂ | 4,5-Dihydro-3-furyl |
| 252 | OCH₂CH₂ | 2,3-Dihydro-3-furyl |
| 253 | OCH₂CH₂ | 2-Thienyl |
| 254 | OCH₂CH₂ | 4,5-Dihydro-2-thienyl |
| 255 | OCH₂CH₂ | 2,3-Dihydro-2-thienyl |
| 256 | OCH₂CH₂ | 5-Chlor-2-thienyl |
| 257 | OCH₂CH₂ | 5-Methyl-2-thienyl |
| 258 | OCH₂CH₂ | 3-Thienyl |
| 259 | OCH₂CH₂ | 4,5-Dihydro-3-thienyl |
| 260 | OCH₂CH₂ | 2,3-Dihydro-3-thienyl |
| 261 | OCH₂CH₂ | 2-Pyrrolyl |
| 262 | OCH₂CH₂ | 2,5-Dihydro-2-pyrrolyl |
| 263 | OCH₂CH₂ | 3-Pyrrolyl |
| 264 | OCH₂CH₂ | 2,5-Dihydro-3-pyrrolyl |
| 265 | OCH₂CH₂ | 3-Isoxazolyl |
| 266 | OCH₂CH₂ | 4-Methyl-3-isoxazolyl |
| 267 | OCH₂CH₂ | 5-Methyl-3-isoxazolyl |
| 268 | OCH₂CH₂ | 4,5-Dimethyl-3-isoxazolyl |
| 269 | OCH₂CH₂ | 4,5-Dihydro-3-isoxazolyl |
| 270 | OCH₂CH₂ | 4-Methyl-4,5-dihydro-3-isoxazolyl |
| 271 | OCH₂CH₂ | 5-Methyl-4,5-dihydro-3-isoxazolyl |
| 272 | OCH₂CH₂ | 4,5-Dimethyl-4,5-di-hydro-3-isoxazolyl |
| 273 | OCH₂CH₂ | 4-Isoxazolyl |
| 274 | OCH₂CH₂ | 3-Methyl-4-isoxazolyl |
| 275 | OCH₂CH₂ | 5-Methyl-4-isoxazolyl |
| 276 | OCH₂CH₂ | 5-Cyclopropyl-4-isoxazolyl |
| 277 | OCH₂CH₂ | 5-Phenyl-4-isoxazolyl |
| 278 | OCH₂CH₂ | 3,5-Dimethyl-4-isoxazolyl |
| 279 | OCH₂CH₂ | 4,5-Dihydro-4-isoxazolyl |
| 280 | OCH₂CH₂ | 3-Methyl-4,5-dihydro-4-isoxazolyl |
| 281 | OCH₂CH₂ | 5-Methyl-4,5-dihydro-4-isoxazolyl |
| 282 | OCH₂CH₂ | 3,5-Dimethyl-4,5-di-hydro-4-isoxazolyl |
| 283 | OCH₂CH₂ | 5-Isoxazolyl |
| 284 | OCH₂CH₂ | 3-Methyl-5-isoxazolyl |
| 285 | OCH₂CH₂ | 4-Methyl-5-isoxazolyl |
| 286 | OCH₂CH₂ | 3,4-Dimethyl-5-isoxazolyl |
| 287 | OCH₂CH₂ | 4,5-Dihydro-5-isoxazolyl |
| 288 | OCH₂CH₂ | 3-Methyl-4,5-dihydro-5-isoxazolyl |
| 289 | OCH₂CH₂ | 4-Methyl-4,5-dihydro-5-isoxazolyl |
| 290 | OCH₂CH₂ | 3,4-Dimethyl-4,5-di-hydro-5-isoxazolyl |
| 291 | OCH₂CH₂ | 3-Isothiazolyl |
| 292 | OCH₂CH₂ | 4-Methyl-3-isothiazolyl |
| 293 | OCH₂CH₂ | 5-Methyl-3-isothiazolyl |
| 294 | OCH₂CH₂ | 4-Isothiazolyl |
| 295 | OCH₂CH₂ | 3-Methyl-4-isothiazolyl |
| 296 | OCH₂CH₂ | 5-Methyl-4-isothiazolyl |
| 297 | OCH₂CH₂ | 5-Isothiazolyl |
| 298 | OCH₂CH₂ | 3-Methyl-5-isothiazolyl |
| 299 | OCH₂CH₂ | 4-Methyl-5-isothiazolyl |
| 300 | OCH₂CH₂ | 2-Oxazolyl |
| 301 | OCH₂CH₂ | 4-Oxazolyl |
| 302 | OCH₂CH₂ | 5-Oxazolyl |
| 303 | OCH₂CH₂ | 2-Thiazolyl |
| 304 | OCH₂CH₂ | 4-Thiazolyl |
| 305 | OCH₂CH₂ | 5-Thiazolyl |
| 306 | OCH₂CH₂ | 3-Pyrazolyl |
| 307 | OCH₂CH₂ | 4-Pyrazolyl |
| 308 | OCH₂CH₂ | 1-Methyl-3-pyrazolyl |
| 309 | OCH₂CH₂ | 1-Methyl-4-pyrazolyl |
| 310 | OCH₂CH₂ | 1-Methyl-5-pyrazolyl |
| 311 | OCH₂CH₂ | 2-Imidazolyl |
| 312 | OCH₂CH₂ | 1-Methyl-2-imidazolyl |
| 313 | OCH₂CH₂ | 5-Methyl-[1,3,4]-2-oxadiazolyl |
| 314 | OCH₂CH₂ | 5-Methyl-[1,2,4]-3-oxadiazolyl |
| 315 | OCH₂CH₂ | 5-Methyl-[1,3,4]-2-thiadiazolyl |
| 316 | OCH₂CH₂ | 5-Methyl-[1,2,4]-3-thiadiazolyl |
| 317 | OCH₂CH₂ | [1,2,4]-3-triazolyl |
| 318 | OCH₂CH₂ | [1,2,3]-4-triazolyl |
| 319 | OCH₂CH₂ | 2-Pyridyl |
| 320 | OCH₂CH₂ | 6-Chlor-2-pyridyl |
| 321 | OCH₂CH₂ | 6-Methoxy-2-pyridyl |
| 322 | OCH₂CH₂ | 6-Trifluormethyl-2-pyridyl |
| 323 | OCH₂CH₂ | 3-Pyridyl |
| 324 | OCH₂CH₂ | 2-Chlor-3-pyridyl |
| 325 | OCH₂CH₂ | 2-Methoxy-3-pyridyl |
| 326 | OCH₂CH₂ | 4-Pyridyl |
| 327 | OCH₂CH₂ | 2-Chlor-4-pyridyl |
| 328 | OCH₂CH₂ | 2-Methoxy-4-pyridyl |
| 329 | OCH₂CH₂ | 2-Ethoxy-4-pyridyl |
| 330 | OCH₂CH₂ | 2-Methylthio-4-pyridyl |
| 331 | OCH₂CH₂ | 2-Trifluormethyl-5-pyridyl |
| 332 | OCH₂CH₂ | 2-Pyrimidinyl |
| 333 | OCH₂CH₂ | 3-Pyrimidinyl |
| 334 | OCH₂CH₂ | 4-Pyrimidinyl |
| 335 | OCH₂CH₂ | 2-Pyrazinyl |
| 336 | OCH₂CH₂ | 3-Pyridazinyl |
| 337 | OCH₂CH₂ | 4-Pyridazinyl |
| 338 | OCH₂CH₂ | 2-(2*H*-1,3-oxazinyl) |
| 339 | OCH₂CH₂ | 2-(6*H*-1,3-oxazinyl) |
| 340 | OCH₂CH₂ | 4-(6*H*-1,3-oxazinyl) |
| 341 | OCH₂CH₂ | 6-(6*H*-1,3-oxazinyl) |
| 342 | OCH₂CH₂ | [1,3,5]-2-Triazinyl |
| 343 | OCH₂CH₂ | [1,2,4]-3-Triazinyl |
| 344 | OCH₂CH₂ | [1,2,4]-5-Triazinyl |
| 345 | OCH₂CH₂ | [1,2,4]-6-Triazinyl |
| 346 | CH₂CH₂O | Oxiranyl |
| 347 | CH₂CH₂O | 3-Methyl-2-oxiranyl |
| 348 | CH₂CH₂O | 2-Oxetanyl |
| 349 | CH₂CH₂O | 3-Hydroxy-3-methyl-2-oxetanyl |
| 350 | CH₂CH₂O | 3-Hydroxy-3-ethyl-2-oxetanyl |
| 351 | CH₂CH₂O | 3-Hydroxy-3-propyl-2-oxetanyl |
| 352 | CH₂CH₂O | 3-Hydroxy-3-butyl-2-oxetanyl |
| 353 | CH₂CH₂O | 3-Methoxy-3-methyl-2-oxetanyl |
| 354 | CH₂CH₂O | 3-Methoxy-3-ethyl-2-oxetanyl |
| 355 | CH₂CH₂O | 3-Methoxy-3-propyl-2-oxetanyl |
| 356 | CH₂CH₂O | 3-Methoxy-3-butyl-2-oxetanyl |
| 357 | CH₂CH₂O | 3-Trimethylsilyl-oxy-3-methyl-2-oxetanyl |
| 358 | CH₂CH₂O | 3-Trimethylsilyl-oxy-3-ethyl-2-oxetanyl |
| 359 | CH₂CH₂O | 3-Trimethylsilyl-oxy-3-propyl-2-oxetanyl |
| 360 | CH₂CH₂O | 3-Trimethylsilyl-oxy-3-butyl-2-oxetanyl |
| 361 | CH₂CH₂O | 3-Oxetanyl |
| 362 | CH₂CH₂O | 2-Furyl |
| 363 | CH₂CH₂O | 4,5-Dihydro-2-furyl |
| 364 | CH₂CH₂O | 2,3-Dihydro-2-furyl |
| 365 | CH₂CH₂O | 3-Furyl |
| 366 | CH₂CH₂O | 4,5-Dihydro-3-furyl |
| 367 | CH₂CH₂O | 2,3-Dihydro-3-furyl |
| 368 | CH₂CH₂O | 2-Thienyl |
| 369 | CH₂CH₂O | 4,5-Dihydro-2-thienyl |
| 370 | CH₂CH₂O | 2,3-Dihydro-2-thienyl |
| 371 | CH₂CH₂O | 5-Chlor-2-thienyl |
| 372 | CH₂CH₂O | 5-Methyl-2-thienyl |
| 373 | CH₂CH₂O | 3-Thienyl |
| 374 | CH₂CH₂O | 4,5-Dihydro-3-thienyl |
| 375 | CH₂CH₂O | 2,3-Dihydro-3-thienyl |
| 376 | CH₂CH₂O | 2-Pyrrolyl |
| 377 | CH₂CH₂O | 2,5-Dihydro-2-pyrrolyl |
| 378 | CH₂CH₂O | 3-Pyrrolyl |
| 379 | CH₂CH₂O | 2,5-Dihydro-3-pyrrolyl |
| 380 | CH₂CH₂O | 3-Isoxazolyl |
| 381 | CH₂CH₂O | 4-Methyl-3-isoxazolyl |
| 382 | CH₂CH₂O | 5-Methyl-3-isoxazolyl |
| 383 | CH₂CH₂O | 4,5-Dimethyl-3-isoxazolyl |
| 384 | CH₂CH₂O | 4,5-Dihydro-3-isoxazolyl |
| 385 | CH₂CH₂O | 4-Methyl-4,5-dihydro-3-isoxazolyl |
| 386 | CH₂CH₂O | 5-Methyl-4,5-dihydro-3-isoxazolyl |
| 387 | CH₂CH₂O | 4,5-Dimethyl-4,5-di-hydro-3-isoxazolyl |
| 388 | CH₂CH₂O | 4-Isoxazolyl |
| 389 | CH₂CH₂O | 3-Methyl-4-isoxazolyl |
| 390 | CH₂CH₂O | 5-Methyl-4-isoxazolyl |
| 391 | CH₂CH₂O | 5-Cyclopropyl-4-isoxazolyl |
| 392 | CH₂CH₂O | 5-Phenyl-4-isoxazolyl |
| 393 | CH₂CH₂O | 3,5-Dimethyl-4-isoxazolyl |
| 394 | CH₂CH₂O | 4,5-Dihydro-4-isoxazolyl |
| 395 | CH₂CH₂O | 3-Methyl-4,5-dihydro-4-isoxazolyl |
| 396 | CH₂CH₂O | 5-Methyl-4,5-dihydro-4-isoxazolyl |
| 397 | CH₂CH₂O | 3,5-Dimethyl-4,5-di-hydro-4-isoxazolyl |
| 398 | CH₂CH₂O | 5-Isoxazolyl |
| 399 | CH₂CH₂O | 3-Methyl-5-isoxazolyl |
| 400 | CH₂CH₂O | 4-Methyl-5-isoxazolyl |
| 401 | CH₂CH₂O | 3,4-Dimethyl-5-isoxazolyl |
| 402 | CH₂CH₂O | 4,5-Dihydro-5-isoxazolyl |
| 403 | CH₂CH₂O | 3-Methyl-4,5-dihydro-5-isoxazolyl |
| 404 | CH₂CH₂O | 4-Methyl-4,5-dihydro-5-isoxazolyl |
| 405 | CH₂CH₂O | 3,4-Dimethyl-4,5-di-hydro-5-isoxazolyl |
| 406 | CH₂CH₂O | 3-Isothiazolyl |
| 407 | CH₂CH₂O | 4-Methyl-3-isothiazolyl |
| 408 | CH₂CH₂O | 5-Methyl-3-isothiazolyl |
| 409 | CH₂CH₂O | 4-Isothiazolyl |
| 410 | CH₂CH₂O | 3-Methyl-4-isothiazolyl |
| 411 | CH₂CH₂O | 5-Methyl-4-isothiazolyl |
| 412 | CH₂CH₂O | 5-Isothiazolyl |
| 413 | CH₂CH₂O | 3-Methyl-5-isothiazolyl |
| 414 | CH₂CH₂O | 4-Methyl-5-isothiazolyl |
| 415 | CH₂CH₂O | 2-Oxazolyl |
| 416 | CH₂CH₂O | 4-Oxazolyl |
| 417 | CH₂CH₂O | 5-Oxazolyl |
| 418 | CH₂CH₂O | 2-Thiazolyl |
| 419 | CH₂CH₂O | 4-Thiazolyl |
| 420 | CH₂CH₂O | 5-Thiazolyl |
| 421 | CH₂CH₂O | 3-Pyrazolyl |
| 422 | CH₂CH₂O | 4-Pyrazolyl |
| 423 | CH₂CH₂O | 1-Methyl-3-pyrazolyl |
| 424 | CH₂CH₂O | 1-Methyl-4-pyrazolyl |
| 425 | CH₂CH₂O | 1-Methyl-5-pyrazolyl |
| 426 | CH₂CH₂O | 2-Imidazolyl |
| 427 | CH₂CH₂O | 1-Methyl-2-imidazolyl |
| 428 | CH₂CH₂O | 5-Methyl-[1,3,4]-2-oxadiazolyl |
| 429 | CH₂CH₂O | 5-Methyl-[1,2,4]-3-oxadiazolyl |
| 430 | CH₂CH₂O | 5-Methyl-[1,3,4]-2-thiadiazolyl |
| 431 | CH₂CH₂O | 5-Methyl-[1,2,4]-3-thiadiazolyl |
| 432 | CH₂CH₂O | [1,2,4]-3-triazolyl |
| 433 | CH₂CH₂O | [1,2,3]-4-triazolyl |
| 434 | CH₂CH₂O | 2-Pyridyl |
| 435 | CH₂CH₂O | 6-Chlor-2-pyridyl |
| 436 | CH₂CH₂O | 6-Methoxy-2-pyridyl |
| 437 | CH₂CH₂O | 6-Trifluormethyl-2-pyridyl |
| 438 | CH₂CH₂O | 3-Pyridyl |
| 439 | CH₂CH₂O | 2-Chlor-3-pyridyl |
| 440 | CH₂CH₂O | 2-Methoxy-3-pyridyl |
| 441 | CH₂CH₂O | 4-Pyridyl |
| 442 | CH₂CH₂O | 2-Chlor-4-pyridyl |
| 443 | CH₂CH₂O | 2-Methoxy-4-pyridyl |
| 444 | CH₂CH₂O | 2-Ethoxy-4-pyridyl |
| 445 | CH₂CH₂O | 2-Methylthio-4-pyridyl |
| 446 | CH₂CH₂O | 2-Trifluormethyl-5-pyridyl |
| 447 | CH₂CH₂O | 2-Pyrimidinyl |
| 448 | CH₂CH₂O | 3-Pyrimidinyl |
| 449 | CH₂CH₂O | 4-Pyrimidinyl |
| 450 | CH₂CH₂O | 2-Pyrazinyl |
| 451 | CH₂CH₂O | 3-Pyridazinyl |
| 452 | CH₂CH₂O | 4-Pyridazinyl |
| 453 | CH₂CH₂O | 2-(2*H*-1,3-oxazinyl) |
| 454 | CH₂CH₂O | 2-(6*H*-1,3-oxazinyl) |
| 455 | CH₂CH₂O | 4-(6*H*-1,3-oxazinyl) |
| 456 | CH₂CH₂O | 6-(6*H*-1,3-oxazinyl) |
| 457 | CH₂CH₂O | [1,3,5]-2-Triazinyl |
| 458 | CH₂CH₂O | [1,2,4]-3-Triazinyl |
| 459 | CH₂CH₂O | [1,2,4]-5-Triazinyl |
| 460 | CH₂CH₂O | [1,2,4]-6-Triazinyl |
| 461 | CH₂OCH₂ | Oxiranyl |
| 462 | CH₂OCH₂ | 3-Methyl-2-oxiranyl |
| 463 | CH₂OCH₂ | 2-Oxetanyl |
| 464 | CH₂OCH₂ | 3-Hydroxy-3-methyl-2-oxetanyl |
| 465 | CH₂OCH₂ | 3-Hydroxy-3-ethyl-2-oxetanyl |
| 466 | CH₂OCH₂ | 3-Hydroxy-3-propyl-2-oxetanyl |
| 467 | CH₂OCH₂ | 3-Hydroxy-3-butyl-2-oxetanyl |
| 468 | CH₂OCH₂ | 3-Methoxy-3-methyl-2-oxetanyl |
| 469 | CH₂OCH₂ | 3-Methoxy-3-ethyl-2-oxetanyl |
| 470 | CH₂OCH₂ | 3-Methoxy-3-propyl-2-oxetanyl |
| 471 | CH₂OCH₂ | 3-Methoxy-3-butyl-2-oxetanyl |
| 472 | CH₂OCH₂ | 3-Trimethylsilyl-oxy-3-methyl-2-oxetanyl |
| 473 | CH₂OCH₂ | 3-Trimethylsilyl-oxy-3-ethyl-2-oxetanyl |
| 474 | CH₂OCH₂ | 3-Trimethylsilyl-oxy-3-propyl-2-oxetanyl |
| 475 | CH₂OCH₂ | 3-Trimethylsilyl-oxy-3-butyl-2-oxetanyl |
| 476 | CH₂OCH₂ | 3-Oxetanyl |
| 477 | CH₂OCH₂ | 2-Furyl |
| 478 | CH₂OCH₂ | 4,5-Dihydro-2-furyl |
| 479 | CH₂OCH₂ | 2,3-Dihydro-2-furyl |
| 480 | CH₂OCH₂ | 3-Furyl |
| 481 | CH₂OCH₂ | 4,5-Dihydro-3-furyl |
| 482 | CH₂OCH₂ | 2,3-Dihydro-3-furyl |
| 483 | CH₂OCH₂ | 2-Thienyl |
| 484 | CH₂OCH₂ | 4,5-Dihydro-2-thienyl |
| 485 | CH₂OCH₂ | 2,3-Dihydro-2-thienyl |
| 486 | CH₂OCH₂ | 5-Chlor-2-thienyl |
| 487 | CH₂OCH₂ | 5-Methyl-2-thienyl |
| 488 | CH₂OCH₂ | 3-Thienyl |
| 489 | CH₂OCH₂ | 4,5-Dihydro-3-thienyl |
| 490 | CH₂OCH₂ | 2,3-Dihydro-3-thienyl |
| 491 | CH₂OCH₂ | 2-Pyrrolyl |
| 492 | CH₂OCH₂ | 2,5-Dihydro-2-pyrrolyl |
| 493 | CH₂OCH₂ | 3-Pyrrolyl |
| 494 | CH₂OCH₂ | 2,5-Dihydro-3-pyrrolyl |
| 495 | CH₂OCH₂ | 3-Isoxazolyl |
| 496 | CH₂OCH₂ | 4-Methyl-3-isoxazolyl |
| 497 | CH₂OCH₂ | 5-Methyl-3-isoxazolyl |
| 498 | CH₂OCH₂ | 4,5-Dimethyl-3-isoxazolyl |
| 499 | CH₂OCH₂ | 4,5-Dihydro-3-isoxazolyl |
| 500 | CH₂OCH₂ | 4-Methyl-4,5-dihydro-3-isoxazolyl |
| 501 | CH₂OCH₂ | 5-Methyl-4,5-dihydro-3-isoxazolyl |
| 502 | CH₂OCH₂ | 4,5-Dimethyl-4,5-di-hydro-3-isoxazolyl |
| 503 | CH₂OCH₂ | 4-Isoxazolyl |
| 504 | CH₂OCH₂ | 3-Methyl-4-isoxazolyl |
| 505 | CH₂OCH₂ | 5-Methyl-4-isoxazolyl |
| 506 | CH₂OCH₂ | 5-Cyclopropyl-4-isoxazolyl |
| 507 | CH₂OCH₂ | 5-Phenyl-4-isoxazolyl |
| 508 | CH₂OCH₂ | 3,5-Dimethyl-4-isoxazolyl |
| 509 | CH₂OCH₂ | 4,5-Dihydro-4-isoxazolyl |
| 510 | CH₂OCH₂ | 3-Methyl-4,5-dihydro-4-isoxazolyl |
| 511 | CH₂OCH₂ | 5-Methyl-4,5-dihydro-4-isoxazolyl |
| 512 | CH₂OCH₂ | 3,5-Dimethyl-4,5-di-hydro-4-isoxazolyl |
| 513 | CH₂OCH₂ | 5-Isoxazolyl |
| 514 | CH₂OCH₂ | 3-Methyl-5-isoxazolyl |
| 515 | CH₂OCH₂ | 4-Methyl-5-isoxazolyl |
| 516 | CH₂OCH₂ | 3,4-Dimethyl-5-isoxazolyl |
| 517 | CH₂OCH₂ | 4,5-Dihydro-5-isoxazolyl |
| 518 | CH₂OCH₂ | 3-Methyl-4,5-dihydro-5-isoxazolyl |
| 519 | CH₂OCH₂ | 4-Methyl-4,5-dihydro-5-isoxazolyl |
| 520 | CH₂OCH₂ | 3,4-Dimethyl-4,5-di-hydro-5-isoxazolyl |
| 521 | CH₂OCH₂ | 3-Isothiazolyl |
| 522 | CH₂OCH₂ | 4-Methyl-3-isothiazolyl |
| 523 | CH₂OCH₂ | 5-Methyl-3-isothiazolyl |
| 524 | CH₂OCH₂ | 4-Isothiazolyl |
| 525 | CH₂OCH₂ | 3-Methyl-4-isothiazolyl |
| 526 | CH₂OCH₂ | 5-Methyl-4-isothiazolyl |
| 527 | CH₂OCH₂ | 5-Isothiazolyl |
| 528 | CH₂OCH₂ | 3-Methyl-5-isothiazolyl |
| 529 | CH₂OCH₂ | 4-Methyl-5-isothiazolyl |
| 530 | CH₂OCH₂ | 2-Oxazolyl |
| 531 | CH₂OCH₂ | 4-Oxazolyl |
| 532 | CH₂OCH₂ | 5-Oxazolyl |
| 533 | CH₂OCH₂ | 2-Thiazolyl |
| 534 | CH₂OCH₂ | 4-Thiazolyl |
| 535 | CH₂OCH₂ | 5-Thiazolyl |
| 536 | CH₂OCH₂ | 3-Pyrazolyl |
| 537 | CH₂OCH₂ | 4-Pyrazolyl |
| 538 | CH₂OCH₂ | 1-Methyl-3-pyrazolyl |
| 539 | CH₂OCH₂ | 1-Methyl-4-pyrazolyl |
| 540 | CH₂OCH₂ | 1-Methyl-5-pyrazolyl |
| 541 | CH₂OCH₂ | 2-Imidazolyl |
| 542 | CH₂OCH₂ | 1-Methyl-2-imidazolyl |
| 543 | CH₂OCH₂ | 5-Methyl-[1,3,4]-2-oxadiazolyl |
| 544 | CH₂OCH₂ | 5-Methyl-[1,2,4]-3-oxadiazolyl |
| 545 | CH₂OCH₂ | 5-Methyl-[1,3,4]-2-thiadiazolyl |
| 546 | CH₂OCH₂ | 5-Methyl-[1,2,4]-3-thiadiazolyl |
| 547 | CH₂OCH₂ | [1,2,4]-3-triazolyl |
| 548 | CH₂OCH₂ | [1,2,3]-4-triazolyl |
| 549 | CH₂OCH₂ | 2-Pyridyl |
| 550 | CH₂OCH₂ | 6-Chlor-2-pyridyl |
| 551 | CH₂OCH₂ | 6-Methoxy-2-pyridyl |
| 552 | CH₂OCH₂ | 6-Trifluormethyl-2-pyridyl |
| 553 | CH₂OCH₂ | 3-Pyridyl |
| 554 | CH₂OCH₂ | 2-Chlor-3-pyridyl |
| 555 | CH₂OCH₂ | 2-Methoxy-3-pyridyl |
| 556 | CH₂OCH₂ | 4-Pyridyl |
| 557 | CH₂OCH₂ | 2-Chlor-4-pyridyl |
| 558 | CH₂OCH₂ | 2-Methoxy-4-pyridyl |
| 559 | CH₂OCH₂ | 2-Ethoxy-4-pyridyl |
| 560 | CH₂OCH₂ | 2-Methylthio-4-pyridyl |
| 561 | CH₂OCH₂ | 2-Trifluormethyl-5-pyridyl |
| 562 | CH₂OCH₂ | 2-Pyrimidinyl |
| 563 | CH₂OCH₂ | 3-Pyrimidinyl |
| 564 | CH₂OCH₂ | 4-Pyrimidinyl |
| 565 | CH₂OCH₂ | 2-Pyrazinyl |
| 566 | CH₂OCH₂ | 3-Pyridazinyl |
| 567 | CH₂OCH₂ | 4-Pyridazinyl |
| 568 | CH₂OCH₂ | 2-(2*H*-1,3-oxazinyl) |
| 569 | CH₂OCH₂ | 2-(6*H*-1,3-oxazinyl) |
| 570 | CH₂OCH₂ | 4-(6*H*-1,3-oxazinyl) |
| 571 | CH₂OCH₂ | 6-(6*H*-1,3-oxazinyl) |
| 572 | CH₂OCH₂ | [1,3,5]-2-Triazinyl |
| 573 | CH₂OCH₂ | [1,2,4]-3-Triazinyl |
| 574 | CH₂OCH₂ | [1,2,4]-5-Triazinyl |
| 575 | CH₂OCH₂ | [1,2,4]-6-Triazinyl |
| 576 | CH₂OCH₂CH=CH | Oxiranyl |
| 577 | CH₂OCH₂CH=CH | 3-Methyl-2-oxiranyl |
| 578 | CH₂OCH₂CH=CH | 2-Oxetanyl |
| 579 | CH₂OCH₂CH=CH | 3-Hydroxy-3-methyl-2-oxetanyl |
| 580 | CH₂OCH₂CH=CH | 3-Hydroxy-3-ethyl-2-oxetanyl |
| 581 | CH₂OCH₂CH=CH | 3-Hydroxy-3-propyl-2-oxetanyl |
| 582 | CH₂OCH₂CH=CH | 3-Hydroxy-3-butyl-2-oxetanyl |
| 583 | CH₂OCH₂CH=CH | 3-Methoxy-3-methyl-2-oxetanyl |
| 584 | CH₂OCH₂CH=CH | 3-Methoxy-3-ethyl-2-oxetanyl |
| 585 | CH₂OCH₂CH=CH | 3-Methoxy-3-propyl-2-oxetanyl |
| 586 | CH₂OCH₂CH=CH | 3-Methoxy-3-butyl-2-oxetanyl |
| 587 | CH₂OCH₂CH=CH | 3-Trimethylsilyl-oxy-3-methyl-2-oxetanyl |
| 588 | CH₂OCH₂CH=CH | 3-Trimethylsilyl-oxy-3-ethyl-2-oxetanyl |
| 589 | CH₂OCH₂CH=CH | 3-Trimethylsilyl-oxy-3-propyl-2-oxetanyl |
| 590 | CH₂OCH₂CH=CH | 3-Trimethylsilyl-oxy-3-butyl-2-oxetanyl |
| 591 | CH₂OCH₂CH=CH | 3-Oxetanyl |
| 592 | CH₂OCH₂CH=CH | 2-Furyl |
| 593 | CH₂OCH₂CH=CH | 4,5-Dihydro-2-furyl |
| 594 | CH₂OCH₂CH=CH | 2,3-Dihydro-2-furyl |
| 595 | CH₂OCH₂CH=CH | 3-Furyl |
| 596 | CH₂OCH₂CH=CH | 4,5-Dihydro-3-furyl |
| 597 | CH₂OCH₂CH=CH | 2,3-Dihydro-3-furyl |
| 598 | CH₂OCH₂CH=CH | 2-Thienyl |
| 599 | CH₂OCH₂CH=CH | 4,5-Dihydro-2-thienyl |
| 600 | CH₂OCH₂CH=CH | 2,3-Dihydro-2-thienyl |
| 601 | CH₂OCH₂CH=CH | 5-Chlor-2-thienyl |
| 602 | CH₂OCH₂CH=CH | 5-Methyl-2-thienyl |
| 603 | CH₂OCH₂CH=CH | 3-Thienyl |
| 604 | CH₂OCH₂CH=CH | 4,5-Dihydro-3-thienyl |
| 605 | CH₂OCH₂CH=CH | 2,3-Dihydro-3-thienyl |
| 606 | CH₂OCH₂CH=CH | 2-Pyrrolyl |
| 607 | CH₂OCH₂CH=CH | 2,5-Dihydro-2-pyrrolyl |
| 608 | CH₂OCH₂CH=CH | 3-Pyrrolyl |
| 609 | CH₂OCH₂CH=CH | 2,5-Dihydro-3-pyrrolyl |
| 610 | CH₂OCH₂CH=CH | 3-Isoxazolyl |
| 611 | CH₂OCH₂CH=CH | 4-Methyl-3-isoxazolyl |
| 612 | CH₂OCH₂CH=CH | 5-Methyl-3-isoxazolyl |
| 613 | CH₂OCH₂CH=CH | 4,5-Dimethyl-3-isoxazolyl |
| 614 | CH₂OCH₂CH=CH | 4,5-Dihydro-3-isoxazolyl |
| 615 | CH₂OCH₂CH=CH | 4-Methyl-4,5-dihydro-3-isoxazolyl |
| 616 | CH₂OCH₂CH=CH | 5-Methyl-4,5-dihydro-3-isoxazolyl |
| 617 | CH₂OCH₂CH=CH | 4,5-Dimethyl-4,5-di-hydro-3-isoxazolyl |
| 618 | CH₂OCH₂CH=CH | 4-Isoxazolyl |
| 619 | CH₂OCH₂CH=CH | 3-Methyl-4-isoxazolyl |
| 620 | CH₂OCH₂CH=CH | 5-Methyl-4-isoxazolyl |
| 621 | CH₂OCH₂CH=CH | 5-Cyclopropyl-4-isoxazolyl |
| 622 | CH₂OCH₂CH=CH | 5-Phenyl-4-isoxazolyl |
| 623 | CH₂OCH₂CH=CH | 3,5-Dimethyl-4-isoxazolyl |
| 624 | CH₂OCH₂CH=CH | 4,5-Dihydro-4-isoxazolyl |
| 625 | CH₂OCH₂CH=CH | 3-Methyl-4,5-dihydro-4-isoxazolyl |
| 626 | CH₂OCH₂CH=CH | 5-Methyl-4,5-dihydro-4-isoxazolyl |
| 627 | CH₂OCH₂CH=CH | 3,5-Dimethyl-4,5-di-hydro-4-isoxazolyl |
| 628 | CH₂OCH₂CH=CH | 5-Isoxazolyl |
| 629 | CH₂OCH₂CH=CH | 3-Methyl-5-isoxazolyl |
| 630 | CH₂OCH₂CH=CH | 4-Methyl-5-isoxazolyl |
| 631 | CH₂OCH₂CH=CH | 3,4-Dimethyl-5-isoxazolyl |
| 632 | CH₂OCH₂CH=CH | 4,5-Dihydro-5-isoxazolyl |
| 633 | CH₂OCH₂CH=CH | 3-Methyl-4,5-dihydro-5-isoxazolyl |
| 634 | CH₂OCH₂CH=CH | 4-Methyl-4,5-dihydro-5-isoxazolyl |
| 635 | CH₂OCH₂CH=CH | 3,4-Dimethyl-4,5-di-hydro-5-isoxazolyl |
| 636 | CH₂OCH₂CH=CH | 3-Isothiazolyl |
| 637 | CH₂OCH₂CH=CH | 4-Methyl-3-isothiazolyl |
| 638 | CH₂OCH₂CH=CH | 5-Methyl-3-isothiazolyl |
| 639 | CH₂OCH₂CH=CH | 4-Isothiazolyl |
| 640 | CH₂OCH₂CH=CH | 3-Methyl-4-isothiazolyl |
| 641 | CH₂OCH₂CH=CH | 5-Methyl-4-isothiazolyl |
| 642 | CH₂OCH₂CH=CH | 5-Isothiazolyl |
| 643 | CH₂OCH₂CH=CH | 3-Methyl-5-isothiazolyl |
| 644 | CH₂OCH₂CH=CH | 4-Methyl-5-isothiazolyl |
| 645 | CH₂OCH₂CH=CH | 2-Oxazolyl |
| 646 | CH₂OCH₂CH=CH | 4-Oxazolyl |
| 647 | CH₂OCH₂CH=CH | 5-Oxazolyl |
| 648 | CH₂OCH₂CH=CH | 2-Thiazolyl |
| 649 | CH₂OCH₂CH=CH | 4-Thiazolyl |
| 650 | CH₂OCH₂CH=CH | 5-Thiazolyl |
| 651 | CH₂OCH₂CH=CH | 3-Pyrazolyl |
| 652 | CH₂OCH₂CH=CH | 4-Pyrazolyl |
| 653 | CH₂OCH₂CH=CH | 1-Methyl-3-pyrazolyl |
| 654 | CH₂OCH₂CH=CH | 1-Methyl-4-pyrazolyl |
| 655 | CH₂OCH₂CH=CH | 1-Methyl-5-pyrazolyl |
| 656 | CH₂OCH₂CH=CH | 2-Imidazolyl |
| 657 | CH₂OCH₂CH=CH | 1-Methyl-2-imidazolyl |
| 658 | CH₂OCH₂CH=CH | 5-Methyl-[1,3,4]-2-oxadiazolyl |
| 659 | CH₂OCH₂CH=CH | 5-Methyl-[1,2,4]-3-oxadiazolyl |
| 660 | CH₂OCH₂CH=CH | 5-Methyl-[1,3,4]-2-thiadiazolyl |
| 661 | CH₂OCH₂CH=CH | 5-Methyl-[1,2,4]-3-thiadiazolyl |
| 662 | CH₂OCH₂CH=CH | [1,2,4]-3-triazolyl |
| 663 | CH₂OCH₂CH=CH | [1,2,3]-4-triazolyl |
| 664 | CH₂OCH₂CH=CH | 2-Pyridyl |
| 665 | CH₂OCH₂CH=CH | 6-Chlor-2-pyridyl |
| 666 | CH₂OCH₂CH=CH | 6-Methoxy-2-pyridyl |
| 667 | CH₂OCH₂CH=CH | 6-Trifluormethyl-2-pyridyl |
| 668 | CH₂OCH₂CH=CH | 3-Pyridyl |
| 669 | CH₂OCH₂CH=CH | 2-Chlor-3-pyridyl |
| 670 | CH₂OCH₂CH=CH | 2-Methoxy-3-pyridyl |
| 671 | CH₂OCH₂CH=CH | 4-Pyridyl |
| 672 | CH₂OCH₂CH=CH | 2-Chlor-4-pyridyl |
| 673 | CH₂OCH₂CH=CH | 2-Methoxy-4-pyridyl |
| 674 | CH₂OCH₂CH=CH | 2-Ethoxy-4-pyridyl |
| 675 | CH₂OCH₂CH=CH | 2-Methylthio-4-pyridyl |
| 676 | CH₂OCH₂CH=CH | 2-Trifluormethyl-5-pyridyl |
| 677 | CH₂OCH₂CH=CH | 2-Pyrimidinyl |
| 678 | CH₂OCH₂CH=CH | 3-Pyrimidinyl |
| 679 | CH₂OCH₂CH=CH | 4-Pyrimidinyl |
| 680 | CH₂OCH₂CH=CH | 2-Pyrazinyl |
| 681 | CH₂OCH₂CH=CH | 3-Pyridazinyl |
| 682 | CH₂OCH₂CH=CH | 4-Pyridazinyl |
| 683 | CH₂OCH₂CH=CH | 2-(2*H*-1,3-oxazinyl) |
| 684 | CH₂OCH₂CH=CH | 2-(6*H*-1,3-oxazinyl) |
| 685 | CH₂OCH₂CH=CH | 4-(6*H*-1,3-oxazinyl) |
| 686 | CH₂OCH₂CH=CH | 6-(6*H*-1,3-oxazinyl) |
| 687 | CH₂OCH₂CH=CH | [1,3,5]-2-Triazinyl |
| 688 | CH₂OCH₂CH=CH | [1,2,4]-3-Triazinyl |
| 689 | CH₂OCH₂CH=CH | [1,2,4]-5-Triazinyl |
| 690 | CH₂OCH₂CH=CH | [1,2,4]-6-Triazinyl |
| 691 | CH=CHCH₂O | Oxiranyl |
| 692 | CH=CHCH₂O | 3-Methyl-2-oxiranyl |
| 693 | CH=CHCH₂O | 2-Oxetanyl |
| 694 | CH=CHCH₂O | 3-Hydroxy-3-methyl-2-oxetanyl |
| 695 | CH=CHCH₂O | 3-Hydroxy-3-ethyl-2-oxetanyl |
| 696 | CH=CHCH₂O | 3-Hydroxy-3-propyl-2-oxetanyl |
| 697 | CH=CHCH₂O | 3-Hydroxy-3-butyl-2-oxetanyl |
| 698 | CH=CHCH₂O | 3-Methoxy-3-methyl-2-oxetanyl |
| 699 | CH=CHCH₂O | 3-Methoxy-3-ethyl-2-oxetanyl |
| 700 | CH=CHCH₂O | 3-Methoxy-3-propyl-2-oxetanyl |
| 701 | CH=CHCH₂O | 3-Methoxy-3-butyl-2-oxetanyl |
| 702 | CH=CHCH₂O | 3-Trimethylsilyl-oxy-3-methyl-2-oxetanyl |
| 703 | CH=CHCH₂O | 3-Trimethylsilyl-oxy-3-ethyl-2-oxetanyl |
| 704 | CH=CHCH₂O | 3-Trimethylsilyl-oxy-3-propyl-2-oxetanyl |
| 705 | CH=CHCH₂O | 3-Trimethylsilyl-oxy-3-butyl-2-oxetanyl |
| 706 | CH=CHCH₂O | 3-Oxetanyl |
| 707 | CH=CHCH₂O | 2-Furyl |
| 708 | CH=CHCH₂O | 4,5-Dihydro-2-furyl |
| 709 | CH=CHCH₂O | 2,3-Dihydro-2-furyl |
| 710 | CH=CHCH₂O | 3-Furyl |
| 711 | CH=CHCH₂O | 4,5-Dihydro-3-furyl |
| 712 | CH=CHCH₂O | 2,3-Dihydro-3-furyl |
| 713 | CH=CHCH₂O | 2-Thienyl |
| 714 | CH=CHCH₂O | 4,5-Dihydro-2-thienyl |
| 715 | CH=CHCH₂O | 2,3-Dihydro-2-thienyl |
| 716 | CH=CHCH₂O | 5-Chlor-2-thienyl |
| 717 | CH=CHCH₂O | 5-Methyl-2-thienyl |
| 718 | CH=CHCH₂O | 3-Thienyl |
| 719 | CH=CHCH₂O | 4,5-Dihydro-3-thienyl |
| 720 | CH=CHCH₂O | 2,3-Dihydro-3-thienyl |
| 721 | CH=CHCH₂O | 2-Pyrrolyl |
| 722 | CH=CHCH₂O | 2,5-Dihydro-2-pyrrolyl |
| 723 | CH=CHCH₂O | 3-Pyrrolyl |
| 724 | CH=CHCH₂O | 2,5-Dihydro-3-pyrrolyl |
| 725 | CH=CHCH₂O | 3-Isoxazolyl |
| 726 | CH=CHCH₂O | 4-Methyl-3-isoxazolyl |
| 727 | CH=CHCH₂O | 5-Methyl-3-isoxazolyl |
| 728 | CH=CHCH₂O | 4,5-Dimethyl-3-isoxazolyl |
| 729 | CH=CHCH₂O | 4,5-Dihydro-3-isoxazolyl |
| 730 | CH=CHCH₂O | 4-Methyl-4,5-dihydro-3-isoxazolyl |
| 731 | CH=CHCH₂O | 5-Methyl-4,5-dihydro-3-isoxazolyl |
| 732 | CH=CHCH₂O | 4,5-Dimethyl-4,5-di-hydro-3-isoxazolyl |
| 733 | CH=CHCH₂O | 4-Isoxazolyl |
| 734 | CH=CHCH₂O | 3-Methyl-4-isoxazolyl |
| 735 | CH=CHCH₂O | 5-Methyl-4-isoxazolyl |
| 736 | CH=CHCH₂O | 5-Cyclopropyl-4-isoxazolyl |
| 737 | CH=CHCH₂O | 5-Phenyl-4-isoxazolyl |
| 738 | CH=CHCH₂O | 3,5-Dimethyl-4-isoxazolyl |
| 739 | CH=CHCH₂O | 4,5-Dihydro-4-isoxazolyl |
| 740 | CH=CHCH₂O | 3-Methyl-4,5-dihydro-4-isoxazolyl |
| 741 | CH=CHCH₂O | 5-Methyl-4,5-dihydro-4-isoxazolyl |
| 742 | CH=CHCH₂O | 3,5-Dimethyl-4,5-di-hydro-4-isoxazolyl |
| 743 | CH=CHCH₂O | 5-Isoxazolyl |
| 744 | CH=CHCH₂O | 3-Methyl-5-isoxazolyl |
| 745 | CH=CHCH₂O | 4-Methyl-5-isoxazolyl |
| 746 | CH=CHCH₂O | 3,4-Dimethyl-5-isoxazolyl |
| 747 | CH=CHCH₂O | 4,5-Dihydro-5-isoxazolyl |
| 748 | CH=CHCH₂O | 3-Methyl-4,5-dihydro-5-isoxazolyl |
| 749 | CH=CHCH₂O | 4-Methyl-4,5-dihydro-5-isoxazolyl |
| 750 | CH=CHCH₂O | 3,4-Dimethyl-4,5-di-hydro-5-isoxazolyl |
| 751 | CH=CHCH₂O | 3-Isothiazolyl |
| 752 | CH=CHCH₂O | 4-Methyl-3-isothiazolyl |
| 753 | CH=CHCH₂O | 5-Methyl-3-isothiazolyl |
| 754 | CH=CHCH₂O | 4-Isothiazolyl |
| 755 | CH=CHCH₂O | 3-Methyl-4-isothiazolyl |
| 756 | CH=CHCH₂O | 5-Methyl-4-isothiazolyl |
| 757 | CH=CHCH₂O | 5-Isothiazolyl |
| 758 | CH=CHCH₂O | 3-Methyl-5-isothiazolyl |
| 759 | CH=CHCH₂O | 4-Methyl-5-isothiazolyl |
| 760 | CH=CHCH₂O | 2-Oxazolyl |
| 761 | CH=CHCH₂O | 4-Oxazolyl |
| 762 | CH=CHCH₂O | 5-Oxazolyl |
| 763 | CH=CHCH₂O | 2-Thiazolyl |
| 764 | CH=CHCH₂O | 4-Thiazolyl |
| 765 | CH=CHCH₂O | 5-Thiazolyl |
| 766 | CH=CHCH₂O | 3-Pyrazolyl |
| 767 | CH=CHCH₂O | 4-Pyrazolyl |
| 768 | CH=CHCH₂O | 1-Methyl-3-pyrazolyl |
| 769 | CH=CHCH₂O | 1-Methyl-4-pyrazolyl |
| 770 | CH=CHCH₂O | 1-Methyl-5-pyrazolyl |
| 771 | CH=CHCH₂O | 2-Imidazolyl |
| 772 | CH=CHCH₂O | 1-Methyl-2-imidazolyl |
| 773 | CH=CHCH₂O | 5-Methyl-[1,3,4]-2-oxadiazolyl |
| 774 | CH=CHCH₂O | 5-Methyl-[1,2,4]-3-oxadiazolyl |
| 775 | CH=CHCH₂O | 5-Methyl-[1,3,4]-2-thiadiazolyl |
| 776 | CH=CHCH₂O | 5-Methyl-[1,2,4]-3-thiadiazolyl |
| 777 | CH=CHCH₂O | [1,2,4]-3-triazolyl |
| 778 | CH=CHCH₂O | [1,2,3]-4-triazolyl |
| 779 | CH=CHCH₂O | 2-Pyridyl |
| 780 | CH=CHCH₂O | 6-Chlor-2-pyridyl |
| 781 | CH=CHCH₂O | 6-Methoxy-2-pyridyl |
| 782 | CH=CHCH₂O | 6-Trifluormethyl-2-pyridyl |
| 783 | CH=CHCH₂O | 3-Pyridyl |
| 784 | CH=CHCH₂O | 2-Chlor-3-pyridyl |
| 785 | CH=CHCH₂O | 2-Methoxy-3-pyridyl |
| 786 | CH=CHCH₂O | 4-Pyridyl |
| 787 | CH=CHCH₂O | 2-Chlor-4-pyridyl |
| 788 | CH=CHCH₂O | 2-Methoxy-4-pyridyl |
| 789 | CH=CHCH₂O | 2-Ethoxy-4-pyridyl |
| 790 | CH=CHCH₂O | 2-Methylthio-4-pyridyl |
| 791 | CH=CHCH₂O | 2-Trifluormethyl-5-pyridyl |
| 792 | CH=CHCH₂O | 2-Pyrimidinyl |
| 793 | CH=CHCH₂O | 3-Pyrimidinyl |
| 794 | CH=CHCH₂O | 4-Pyrimidinyl |
| 795 | CH=CHCH₂O | 2-Pyrazinyl |
| 796 | CH=CHCH₂O | 3-Pyridazinyl |
| 797 | CH=CHCH₂O | 4-Pyridazinyl |
| 798 | CH=CHCH₂O | 2-(2*H*-1,3-oxazinyl) |
| 799 | CH=CHCH₂O | 2-(6*H*-1,3-oxazinyl) |
| 800 | CH=CHCH₂O | 4-(6*H*-1,3-oxazinyl) |
| 801 | CH=CHCH₂O | 6-(6*H*-1,3-oxazinyl) |
| 802 | CH=CHCH₂O | [1,3,5]-2-Triazinyl |
| 803 | CH=CHCH₂O | [1,2,4]-3-Triazinyl |
| 804 | CH=CHCH₂O | [1,2,4]-5-Triazinyl |
| 805 | CH=CHCH₂O | [1,2,4]-6-Triazinyl |
| 806 | C≡C-CH₂O | Oxiranyl |
| 807 | C≡C-CH₂O | 3-Methyl-2-oxiranyl |
| 808 | C≡C-CH₂O | 2-Oxetanyl |
| 809 | C≡C-CH₂O | 3-Hydroxy-3-methyl-2-oxetanyl |
| 810 | C≡C-CH₂O | 3-Hydroxy-3-ethyl-2-oxetanyl |
| 811 | C≡C-CH₂O | 3-Hydroxy-3-propyl-2-oxetanyl |
| 812 | C≡C-CH₂O | 3-Hydroxy-3-butyl-2-oxetanyl |
| 813 | C≡C-CH₂O | 3-Methoxy-3-methyl-2-oxetanyl |
| 814 | C≡C-CH₂O | 3-Methoxy-3-ethyl-2-oxetanyl |
| 815 | C≡C-CH₂O | 3-Methoxy-3-propyl-2-oxetanyl |
| 816 | C≡C-CH₂O | 3-Methoxy-3-butyl-2-oxetanyl |
| 817 | C≡C-CH₂O | 3-Trimethylsilyl-oxy-3-methyl-2-oxetanyl |
| 818 | C≡C-CH₂O | 3-Trimethylsilyl-oxy-3-ethyl-2-oxetanyl |
| 819 | C≡C-CH₂O | 3-Trimethylsilyl-oxy-3-propyl-2-oxetanyl |
| 820 | C≡C-CH₂O | 3-Trimethylsilyl-oxy-3-butyl-2-oxetanyl |
| 821 | C≡C-CH₂O | 3-Oxetanyl |
| 822 | C≡C-CH₂O | 2-Furyl |
| 823 | C≡C-CH₂O | 4,5-Dihydro-2-furyl |
| 824 | C≡C-CH₂O | 2,3-Dihydro-2-furyl |
| 825 | C≡C-CH₂O | 3-Furyl |
| 826 | C≡C-CH₂O | 4,5-Dihydro-3-furyl |
| 827 | C≡C-CH₂O | 2,3-Dihydro-3-furyl |
| 828 | C≡C-CH₂O | 2-Thienyl |
| 829 | C≡C-CH₂O | 4,5-Dihydro-2-thienyl |
| 830 | C≡C-CH₂O | 2,3-Dihydro-2-thienyl |
| 831 | C≡C-CH₂O | 5-Chlor-2-thienyl |
| 832 | C≡C-CH₂O | 5-Methyl-2-thienyl |
| 833 | C≡C-CH₂O | 3-Thienyl |
| 834 | C≡C-CH₂O | 4,5-Dihydro-3-thienyl |
| 835 | C≡C-CH₂O | 2,3-Dihydro-3-thienyl |
| 836 | C≡C-CH₂O | 2-Pyrrolyl |
| 837 | C≡C-CH₂O | 2,5-Dihydro-2-pyrrolyl |
| 838 | C≡C-CH₂O | 3-Pyrrolyl |
| 839 | C≡C-CH₂O | 2,5-Dihydro-3-pyrrolyl |
| 840 | C≡C-CH₂O | 3-Isoxazolyl |
| 841 | C≡C-CH₂O | 4-Methyl-3-isoxazolyl |
| 842 | C≡C-CH₂O | 5-Methyl-3-isoxazolyl |
| 843 | C≡C-CH₂O | 4,5-Dimethyl-3-isoxazolyl |
| 844 | C≡C-CH₂O | 4,5-Dihydro-3-isoxazolyl |
| 845 | C≡C-CH₂O | 4-Methyl-4,5-dihydro-3-isoxazolyl |
| 846 | C≡C-CH₂O | 5-Methyl-4,5-dihydro-3-isoxazolyl |
| 847 | C≡C-CH₂O | 4,5-Dimethyl-4,5-di-hydro-3-isoxazolyl |
| 848 | C≡C-CH₂O | 4-Isoxazolyl |
| 849 | C≡C-CH₂O | 3-Methyl-4-isoxazolyl |
| 850 | C≡C-CH₂O | 5-Methyl-4-isoxazolyl |
| 851 | C≡C-CH₂O | 5-Cyclopropyl-4-isoxazolyl |
| 852 | C≡C-CH₂O | 5-Phenyl-4-isoxazolyl |
| 853 | C≡C-CH₂O | 3,5-Dimethyl-4-isoxazolyl |
| 854 | C≡C-CH₂O | 4,5-Dihydro-4-isoxazolyl |
| 855 | C≡C-CH₂O | 3-Methyl-4,5-dihydro-4-isoxazolyl |
| 856 | C≡C-CH₂O | 5-Methyl-4,5-dihydro-4-isoxazolyl |
| 857 | C≡C-CH₂O | 3,5-Dimethyl-4,5-di-hydro-4-isoxazolyl |
| 858 | C≡C-CH₂O | 5-Isoxazolyl |
| 859 | C≡C-CH₂O | 3-Methyl-5-isoxazolyl |
| 860 | C≡C-CH₂O | 4-Methyl-5-isoxazolyl |
| 861 | C≡C-CH₂O | 3,4-Dimethyl-5-isoxazolyl |
| 862 | C≡C-CH₂O | 4,5-Dihydro-5-isoxazolyl |
| 863 | C≡C-CH₂O | 3-Methyl-4,5-dihydro-5-isoxazolyl |
| 864 | C≡C-CH₂O | 4-Methyl-4,5-dihydro-5-isoxazolyl |
| 865 | C≡C-CH₂O | 3,4-Dimethyl-4,5-di-hydro-5-isoxazolyl |
| 866 | C≡C-CH₂O | 3-Isothiazolyl |
| 867 | C≡C-CH₂O | 4-Methyl-3-isothiazolyl |
| 868 | C≡C-CH₂O | 5-Methyl-3-isothiazolyl |
| 869 | C≡C-CH₂O | 4-Isothiazolyl |
| 870 | C≡C-CH₂O | 3-Methyl-4-isothiazolyl |
| 871 | C≡C-CH₂O | 5-Methyl-4-isothiazolyl |
| 872 | C≡C-CH₂O | 5-Isothiazolyl |
| 873 | C≡C-CH₂O | 3-Methyl-5-isothiazolyl |
| 874 | C≡C-CH₂O | 4-Methyl-5-isothiazolyl |
| 875 | C≡C-CH₂O | 2-Oxazolyl |
| 876 | C≡C-CH₂O | 4-Oxazolyl |
| 877 | C≡C-CH₂O | 5-Oxazolyl |
| 878 | C≡C-CH₂O | 2-Thiazolyl |
| 879 | C≡C-CH₂O | 4-Thiazolyl |
| 880 | C≡C-CH₂O | 5-Thiazolyl |
| 881 | C≡C-CH₂O | 3-Pyrazolyl |
| 882 | C≡C-CH₂O | 4-Pyrazolyl |
| 883 | C≡C-CH₂O | 1-Methyl-3-pyrazolyl |
| 884 | C≡C-CH₂O | 1-Methyl-4-pyrazolyl |
| 885 | C≡C-CH₂O | 1-Methyl-5-pyrazolyl |
| 886 | C≡C-CH₂O | 2-Imidazolyl |
| 887 | C≡C-CH₂O | 1-Methyl-2-imidazolyl |
| 888 | C≡C-CH₂O | 5-Methyl-[1,3,4]-2-oxadiazolyl |
| 889 | C≡C-CH₂O | 5-Methyl-[1,2,4]-3-oxadiazolyl |
| 890 | C≡C-CH₂O | 5-Methyl-[1,3,4]-2-thiadiazolyl |
| 891 | C≡C-CH₂O | 5-Methyl-[1,2,4]-3-thiadiazolyl |
| 892 | C≡C-CH₂O | [1,2,4]-3-triazolyl |
| 893 | C≡C-CH₂O | [1,2,3]-4-triazolyl |
| 894 | C≡C-CH₂O | 2-Pyridyl |
| 895 | C≡C-CH₂O | 6-Chlor-2-pyridyl |
| 896 | C≡C-CH₂O | 6-Methoxy-2-pyridyl |
| 897 | C≡C-CH₂O | 6-Trifluormethyl-2-pyridyl |
| 898 | C≡C-CH₂O | 3-Pyridyl |
| 899 | C≡C-CH₂O | 2-Chlor-3-pyridyl |
| 900 | C≡C-CH₂O | 2-Methoxy-3-pyridyl |
| 901 | C≡C-CH₂O | 4-Pyridyl |
| 902 | C≡C-CH₂O | 2-Chlor-4-pyridyl |
| 903 | C≡C-CH₂O | 2-Methoxy-4-pyridyl |
| 904 | C≡C-CH₂O | 2-Ethoxy-4-pyridyl |
| 905 | C≡C-CH₂O | 2-Methylthio-4-pyridyl |
| 906 | C≡C-CH₂O | 2-Trifluormethyl-5-pyridyl |
| 907 | C≡C-CH₂O | 2-Pyrimidinyl |
| 908 | C≡C-CH₂O | 3-Pyrimidinyl |
| 909 | C≡C-CH₂O | 4-Pyrimidinyl |
| 910 | C≡C-CH₂O | 2-Pyrazinyl |
| 911 | C≡C-CH₂O | 3-Pyridazinyl |
| 912 | C≡C-CH₂O | 4-Pyridazinyl |
| 913 | C≡C-CH₂O | 2-(2*H*-1,3-oxazinyl) |
| 914 | C≡C-CH₂O | 2-(6*H*-1,3-oxazinyl) |
| 915 | C≡C-CH₂O | 4-(6*H*-1,3-oxazinyl) |
| 916 | C≡C-CH₂O | 6-(6*H*-1,3-oxazinyl) |
| 917 | C≡C-CH₂O | [1,3,5]-2-Triazinyl |
| 918 | C≡C-CH₂O | [1,2,4]-3-Triazinyl |
| 919 | C≡C-CH₂O | [1,2,4]-5-Triazinyl |
| 920 | C≡C-CH₂O | [1,2,4]-6-Triazinyl |

| | | |
|---|---|---|
| * Das Brückenglied X¹ ist am linken Ende mit dem zentralen Phenylring und am rechten Ende mit Het verknüpft. | | |

Die folgenden Tabellen 1 - 36 basieren auf den 2-Benzoyl-cyclohexan-1,3-dionen der Formel Ib

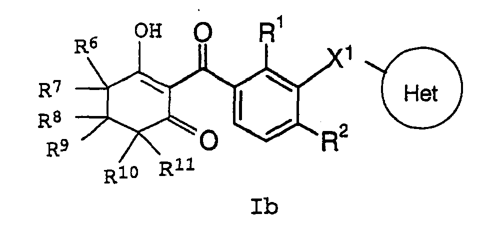

### Tabelle 1: Verbindungen 1.1-1.920

Verbindungen der allgemeinen Formel Ib, in der R¹ Chlor, R² Methylsulfonyl, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 2: Verbindungen 2.1-2.920

Verbindungen der allgemeinen Formel Ib, in der R¹ und R² Chlor, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 3: Verbindungen 3.1-3.920

Verbindungen der allgemeinen Formel Ib, in der R¹ Chlor, R² Trifluormethyl, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 4: Verbindungen 4.1-4.920

Verbindungen der allgemeinen Formel Ib, in der R¹ Methyl, R² Chlor, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 5: Verbindungen 5.1-5.920

Verbindungen der allgemeinen Formel Ib, in der R¹ Methyl, R² Methylsulfonyl, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 6: Verbindungen 6.1-6.920

Verbindungen der allgemeinen Formel Ib, in der R¹ Methyl, R² Trifluormethyl, R⁶, R⁷, R⁸, R⁹, R¹⁰ und R¹¹ Wasserstoff bedeutet und die Substituenten X¹ und Het für jede einzelne Verbindung jeweils einer Zeile der Tabelle A entsprechen.

### Tabelle 7: Verbindungen 7.1-7.920

Verbindungen der allgemeinen Formel Ib, in der R¹ Chlor, R² Methylsulfonyl, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 8: Verbindungen 8.1-8.920

Verbindungen der allgemeinen Formel Ib, in der R¹ und R² Chlor, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 9: Verbindungen 9.1-9.920

Verbindungen der allgemeinen Formel Ib, in der R¹ Chlor, R² Trifluormethyl, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 10: Verbindungen 10.1-10.920

Verbindungen der allgemeinen Formel Ib, in der R¹ Methyl, R² Chlor, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 11: Verbindungen 11.1-11.920

Verbindungen der allgemeinen Formel Ib, in der R¹ Methyl, R² Methylsulfonyl, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 12: Verbindungen 12.1-12.920

Verbindungen der allgemeinen Formel Ib, in der R¹ Methyl, R² Trifluormethyl, R⁶, R⁷, R¹⁰ und R¹¹ Wasserstoff, R⁸ und R⁹ Methyl bedeutet und die Substituenten X¹ und Het für jede einzelne Verbindung jeweils einer Zeile der Tabelle A entsprechen.

### Tabelle 13: Verbindungen 13.1-13.920

Verbindungen der allgemeinen Formel Ib, in der R¹ Chlor, R² Methylsulfonyl, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 14: Verbindungen 14.1-14.920

Verbindungen der allgemeinen Formel Ib, in der R¹ und R² Chlor, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 15: Verbindungen 15.1-15.920

Verbindungen der allgemeinen Formel Ib, in der R¹ Chlor, R² Trifluormethyl, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 16: Verbindungen 16.1-16.920

Verbindungen der allgemeinen Formel Ib, in der R¹ Methyl, R² Chlor, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 17: Verbindungen 17.1-17.920

Verbindungen der allgemeinen Formel Ib, in der R¹ Methyl, R² Methylsulfonyl, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 18: Verbindungen 18.1-18.920

Verbindungen der allgemeinen Formel Ib, in der R¹ Methyl, R² Trifluormethyl, R⁶, R⁷, R⁸ und R⁹ Wasserstoff, R¹⁰ und R¹¹ Methyl bedeutet und die Substituenten X¹ und Het für jede einzelne Verbindung jeweils einer Zeile der Tabelle A entsprechen.

### Tabelle 19: Verbindungen 19.1-19.920

Verbindungen der allgemeinen Formel Ib, in der R¹ Chlor, R² Methylsulfonyl, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 20: Verbindungen 20.1-20.920

Verbindungen der allgemeinen Formel Ib, in der R¹ und R² Chlor, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 21: Verbindungen 21.1-21.920

Verbindungen der allgemeinen Formel Ib, in der R¹ Chlor, R² Trifluormethyl, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 22: Verbindungen 22.1-22.920

Verbindungen der allgemeinen Formel Ib, in der R¹ Methyl, R² Chlor, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 23: Verbindungen 23.1-23.920

Verbindungen der allgemeinen Formel Ib, in der R¹ Methyl, R² Methylsulfonyl, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 24: Verbindungen 24.1-24.920

Verbindungen der allgemeinen Formel Ib, in der R¹ Methyl, R² Trifluormethyl, R⁶, R⁷, R¹⁰ und R¹¹ Methyl bedeutet, die CR⁸R⁹-Einheit eine Gruppe C=O bildet und die Substituenten X¹ und Het für jede einzelne Verbindung jeweils einer Zeile der Tabelle A entsprechen.

### Tabelle 25: Verbindungen 25.1-25.920

Verbindungen der allgemeinen Formel Ib, in der R¹ Methylsulfonyl, R⁶, R⁷, R⁸, R¹⁰ und R¹¹ Wasserstoff, R⁹ Methyl bedeutet, die Substituenten X¹ und Het für jede einzelne Verbindung jeweils einer Zeile der Tabelle A entsprechen.

### Tabelle 26: Verbindungen 26.1-26.920

Verbindungen der allgemeinen Formel Ib, in der R¹ und R² Chlor, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 27: Verbindungen 27.1-27.920

Verbindungen der allgemeinen Formel Ib, in der R¹ Chlor, R² Trifluormethyl, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 28: Verbindungen 28.1-28.920

Verbindungen der allgemeinen Formel Ib, in der R¹ Methyl, R² Chlor, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 29: Verbindungen 29.1-29.920

Verbindungen der allgemeinen Formel Ib, in der R¹ Methyl, R² Methylsulfonyl, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 30: Verbindungen 30.1-30.920

Verbindungen der allgemeinen Formel Ib, in der R¹ Methyl, R² Trifluormethyl, R⁶, R⁷, R⁸, R¹⁰ und R¹¹ Wasserstoff, R⁹ Methyl bedeutet und die Substituenten X¹ und Het für jede einzelne Verbindung jeweils einer Zeile der Tabelle A entsprechen.

### Tabelle 31: Verbindungen 31.1-31.920

Verbindungen der allgemeinen Formel Ib, in der R¹ Chlor, R² Methylsulfonyl, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 32: Verbindungen 32.1-32.920

Verbindungen der allgemeinen Formel Ib, in der R¹ und R² Chlor, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 33: Verbindungen 33.1-33.920

Verbindungen der allgemeinen Formel Ib, in der R¹ Chlor, R² Trifluormethyl, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 34: Verbindungen 34.1-34.920

Verbindungen der allgemeinen Formel Ib, in der R¹ Methyl, R² Chlor, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 35: Verbindungen 35.1-35.920

Verbindungen der allgemeinen Formel Ib, in der R¹ Methyl, R² Methylsulfonyl, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

### Tabelle 36: Verbindungen 36.1-36.920

Verbindungen der allgemeinen Formel Ib, in der R¹ Methyl, R² Trifluormethyl, R⁶, R⁷, R⁹ und R¹⁰ Wasserstoff bedeutet, R⁸ und R¹¹ zusammen eine Methylengruppe bilden und die Substituenten X¹ und Het für jede einzelne Verbindung jeweils einer Zeile der Tabelle A entsprechen.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sätivum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, vernebeln, Verstäuben, Verstreuen oder Gie-Ben angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die substituierte 2-Benzoylcyclohexan-1,3-dione als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentration der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, minestens eines Wirkstoffs. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I 20 Gewichtsteile der Verbindung I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
II 20 Gewichtsteile der Verbindung I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
III 20 Gewichtsteile der Verbindung I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV 20 Gewichtsteile der Verbindung I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V 3 Gewichtsteile der Verbindung I werden mit 97 Gewichtsteilen feinteiligem Kaolin vermischt. Man erhält auf diese Weise ein Stäubemittel, das 3 Gew.-% des Wirkstoffs enthält.
VI 20 Gewichtsteile der Verbindung I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII 1 Gewichtsteil der Verbindung I wird in einer Mischung gelöst, die aus 70 Gewichtsteilen Cyclohexanon, 20 Gewichtsteilen ethoxyliertem Isooctylphenol und 10 Gewichtsteilen ethoxyliertem Ricinusöl besteht. Man erhält ein stabiles Emulsionskonzentrat.
VIII 1 Gewichtsteil der Verbindung I wird in einer Mischung gelöst, die aus 80 Gewichtsteilen Cyclohexanon und 20 Gewichtsteilen Wettol ® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die substituierte 2-Benzoylcyclohexan-1,3-dione mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazone, 2-Aroyl-1,3-cyclohexandione, Hetaryl-Aryl-Ketone, Benzylisooxazolidinone, Meta-CF₃-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.). Nachfolgend werden die Synthesen einiger Edukte und Produkte beschrieben.

{2-Chlor-3-[(1-methylpyrazol-5-yl)oxymethyl]-4-methylsulfonyl-phenyl}-{5,5-dimethyl-1,3-dioxo-cyclohex-2-yl}methanon
Stufe a: 2-Chlor-3-brommethyl-4-methylsulfonyl-benzoesäuremethylester
80 g (0,3 mol) 2-Chlor-3-methyl-4-methylsulfonyl-benzoesäuremethylester werden in 1 1 Tetrachlormethan mit 54 g (0,31 mol) N-Bromsuccinimid und 1,5 g Azoisobutyronitril 6 h auf 76°C erwärmt. Das Reaktionsgemisch wird filtriert und i. Vak. von Lösungsmittel befreit. Ausbeute 104 g; Fp. 83-85°C
Stufe b: 2-Chlor-3-[(1-methylpyrazol-5-yl)oxymethyl]-4-methylsulfonyl-benzoesäuremethylester
4,3 g (44 mmol) 1-Methyl-5-hydroxypyrazol und 9,1 g Kaliumcarbonat werden in 100 ml Tetrahydrofuran 1 h auf 65°C erwärmt und anschließend mit 15 g (44 mmol)2-Chlor-3-brommethyl-4-methylsulfonyl-benzoesäuremethylester in 150 ml Tetrahydrofuran 4 h auf 40°C erhitzt. Nach 12 h Rühren bei Raumtemperatur wird das Lösungsmittel i. Vak. entfernt, in Essigsäureethylester aufgenommen, mit Natriumhydrogencarbonatlösung und Wasser gewaschen, getrocknet und das Lösungsmittel entfernt. Das Rohprodukt wird an Kieselgel chromatographiert (Eluent: Cyclohexan/Essigsäureethylester=1/1).
Ausbeute: 7,6 g; Fp. 70°C
Stufe c: 2-Chlor-3-[(1-methylpyrazol-5-yl)oxymethyl]-4-methylsulfonyl-benzoesäure
6,95 g (19 mmol) 2-Chlor-3-[(1-methylpyrazol-5-yl)oxymethyl]-4-methylsulfonyl-benzoesäuremethylester werden in einen gemisch von 30 ml Tetrahydrofuran und 30 ml Wasser bei Raumtermperatur 12 h mit 0,93 g Lithiumhydroxid behandelt. Das Reaktionsgemisch wird mit 10%-iger Salzsäure auf pH 4 gestellt und mit Methylenchlorid extrahiert. Die vereinigten organischen Phasen werden getrocknet und das Lösungsmittel entfernt. Ausbeute: 4,2 g; Fp. 197°C
Stufe d: {2-Chlor-3-[(1-methylpyrazol-5-yl)oxymethyl]-4-methysulfonyl-phenyl}-{5,5-dimethyl-1,3-dioxocyclohex-2-yl}methanon 1,0 g (2,9 mmol) 2-Chlor-3-[(1-methylpyrazol-5-yl)oxymethyl]-4-methylsulfonyl-benzoesäure, 0,4 g (2,9 mmol) Dimedon und 0,72 g N,N-Dicyclohexylcarbodiimid werden in 50 ml Acetonitril 4 h auf 40°C erwärmt. Nach 12 h Rühren bei Raumtermperatur werden 0,87 g Triethylamin und 0,57 g Triemethylsilylcyanid zugegeben. Anschließend wird 6 h auf 40°C erwärmt, dann abfiltriert, das Lösungsmittel i. Vak. entfernt und der Rückstand an Kieselgel chromatrographiert (Eluent: Toluol/Tetrahydrofuran/Essigsäure: 100/0/0 → 4/1/0,1). Ausbeute: 0,25 g; Fp. 82°C

**Tabelle 37**

| | | | | | | | | | | |
|---|---|---|---|---|---|---|---|---|---|---|
| 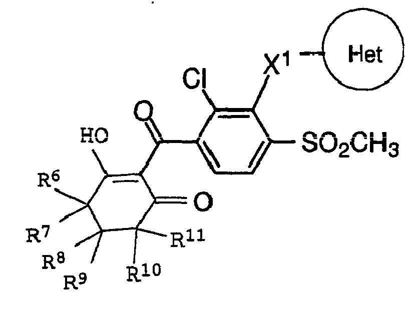 | | | | | | | | | | |

| Nr. | R⁶ | R⁷ | R⁸ | R⁹ | R¹⁰ | R¹¹ | X¹ | Het | Fp. [°C] | ¹H-NMR [ppm] |
|---|---|---|---|---|---|---|---|---|---|---|
| 37.1 | H | H | CH₃ | CH₃ | H | H | CH₂O | 1-Pyrazolyl | 82 | |
| 37.2 | H | H | CH₃ | CH₃ | H | H | CH₂O | 3,5-Dimethyl -1-pyrazolyl | 76 | |
| 37.3 | H | H | CH₃ | CH₃ | H | H | CH₂O | 4-Chlor-1- pyrazolyl | 75 | |
| 37.4 | H | CH₃ | H | H | H | CH₃ | CH₂O | 3,5-Dimethyl -1-pyrazolyl | 74 | |
| 37.5 | H | CH₃ | H | H | H | CH₃ | CH₂O | 4-Chlor-1- pyrazolyl | 79 | |
| 37.6 | CH₃ | CH₃ | C=O | | CH₃ | CH₃ | CH₂O | 3,5-Dimethyl -1-pyrazolyl | 137 | |
| 37.7 | CH₃ | CH₃ | C=O | | CH₃ | CH₃ | CH₂O | 4-Chlor-1- pyrazolyl | 95 | |

### Anwendungsbeispiele

Die herbizide Wirkung der substituierten 2-Benzoyl-cyclohexan-1,3-dione der Formel I ließ sich durch Gewächshausversuche zeigen:
Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

## Patentansprüche

1. 2-Benzoyl-cyclohexan-1,3-dione der Formel I 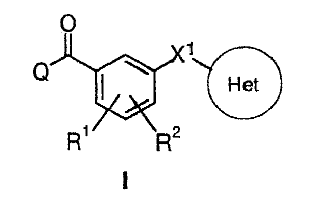 in der die Substituenten folgende Bedeutung haben:
R¹, R² Wasserstoff, Mercapto, Nitro, Halogen, Cyano, Rhodano, C₁-C₆-Alkyl, C₁-C₆-Halogenalkyl, C₁-C₆-Alkoxy, C₂-C₆-Alkenyl, C₂-C₆-Alkinyl, -OR³, -OCOR³, -OSO₂R³, -S(O)nR³, -SO₂OR³, -SO₂N(R³)₂, -NR³SO₂R³ oder -NR³COR³;
R³ Wasserstoff,C₁-C₆-Alkyl, C₁-C₆-Halogenalkyl, C₂-C₆-Alkenyl, C₂-C₆-Alkinyl, Phenyl oder Phenyl-C₁-C₆-alkyl; wobei die genannten Alkylreste partiell oder vollständig halogeniert sein können und/ oder eine bis drei der folgenden Gruppen tragen können:
Hydroxy, Mercapto, Amino, Cyano, R³, -OR³, -SR³, -N(R³)₂, =NOR³, -OCOR³, -SCOR³, -NR³COR³, -CO₂R³, -COSR³, -CON(R³)₂, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei Heterocyclyl, sowie die Heterocyclylreste in Heterocycloxy:
drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, und
Hetaryl, sowie die Hetarylreste in Hetaryloxy:
Aromatische mono- oder polycyclische Reste, welche neben Kohlenstoffringgliedem zusätzlich ein bis vier Stickstoffatome oder ein bis drei Stickstoffatome und ein Sauerstoff- oder ein Schwefelatom oder ein Sauerstoff- und ein Schwefelatom enthalten können, bedeuten und wobei die acht letztgenannten Gruppen durch ein bis drei Halogenatome und/oder einen oder zwei Reste aus folgender Gruppe: Nitro, Cyano, Methyl, Trifluormethyl, Methoxy, Trifluormethoxy oder Methoxycarbonyl substituiert sein können;
n 0, 1 oder 2;
Q ein gegebenenfalls substituierter, in 2-Stellung verknüpfter Cyclohexan-1,3-dion-Ring der Formel II, 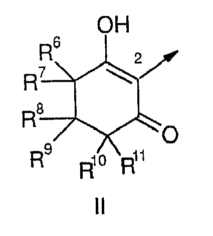 ist, wobei R⁶, R⁷, R⁹ und R¹¹ für Wasserstoff oder C₁-C₄-Alkyl stehen;
R⁸ für Wasserstoff, C₁-C₄-Alkyl oder C₃-C₄-Cycloalkyl steht, wobei die beiden letztgenannten Gruppen einen bis drei der folgenden Substituenten tragen können:
Halogen,C₁-C₄-Alkylthio oder C₁-C₄-Alkoxy;
oder
für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch einen bis drei C₁-C₄-Alkylreste substituiert sein können;
R¹⁰ für Wasserstoff, C₁-C₄-Alkyl oder C₁-C₆-Alkoxycarbonyl steht;
oder
R⁸ und R¹¹ gemeinsam eine π-Bindung oder einen drei- bis sechsgliedrigen carbocyclischen Ring bilden;
oder
die CR⁸R⁹-Einheit durch C=O ersetzt sein kann;
X¹ eine geradkettige oder verzweigte C₁-C₆-Alkylen-, eine C₂-C₆-Alkenylen- oder eine C₂-C₆-Alkinylenkette, die durch ein Heteroatom ausgewählt aus der Gruppe:
Sauerstoff oder Schwefel
unterbrochen ist und wobei die genannten Alkyl-,
Alkenyl- oder Alkinylreste partiell halogeniert sein können und/oder eine bis drei der folgenden Gruppen tragen können:
-OR⁴, -OCOR⁴, -OCONHR⁴ oder -OSO₂R⁴;
R⁴ Wasserstoff, C₁-C₆-Alkyl, C₂-C₆-Alkenyl,
C₂-C₆-Alkinyl, Phenyl, Phneyl-C₁-C₆-alkyl, wobei die genannten Alkyl-, Alkenyl oder Alkinylreste partiell
oder vollständig halogeniert sein können und/ oder
durch einen oder mehrere der folgenden Reste substituiert sein können:
Hydroxy, Mercapto, Amino, Cyano, Nitro, Formyl, C₁-C₄-Alkylamino, C₁-C₄-Dialkylamino, C₁-C₄-Alkoxy- carbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogen-alkoxy;
Het eine drei- bis sechsgliedrige, teilweise oder vollständig gesättigte, heterocyclische Gruppe, die ein Heteroatom ausgewählt aus einer Gruppe bestehend aus Sauerstoff, Stickstoff und Schwefel enthält,
wobei die genannte heterocyclische Gruppe partiell oder vollständig halogeniert sein kann und/oder durch R substituiert sein kann;
R⁵ Wasserstoff, Hydroxy, Mercapto, Amino, Cyano, Nitro, Formyl, C₁-C₄-Alkylamino, C₁-C₄-Dialkylanüno, C₁-C₄-Alkoxycarbonyl, C₁-C₄-Alkylcarbonyl, C₁-C₄-Alkylcarbonyloxy, C₁-C₄-Alkyl, C₁-C₄-Halogenalkyl, C₁-C₄-Alkylthio, C₁-C₄-Halogenalkylthio, C₁-C₄-Alkoxy, C₁-C₄-Halogenalkoxy, wobei die Alkylreste in allen Fällen jeweils durch einen oder mehrere der folgenden Reste substituiert sein können:
Cyano, Formyl, C₁-C₄-Alkylamino, C₁-C₄-Dialkylamino, C₁-C₄-Alkoxycarbonyl, C₁-C₄-Alkylcarbonyl, C₁-C₄-Alkylcarbonyloxy, C₁-C₄-Alkyl, C₁-C₄-HalogenAlkyl, C₁-C₄-Alkylthio, C₁-C₄-Halogenalkylthio, C₁-C₄-Alkoxy, C₁-C₄-Halogenalkoxy;
sowie deren landwirtschaftlich brauchbaren Salze.

2. 2-Benzoyl-cyclohexan-1,3-dione der Formel I nach Anspruch 1, in der
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR³ oder -S(O)ₙR³ bedeutet;
R² für Wasserstoff oder einen wie voranstehend unter R¹ genannten Rest steht.

3. 4-Benzoyl-cyclohexan-1,3-dione der Formel Ia nach einem der Ansprüche 1 oder 2, 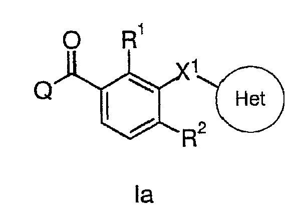 in der die Substituenten R¹, R², Q, X und Het die unter Anspruch 1 genannte Bedeutung haben.

4. 2-Benzoyl-cyclohexan-1,3-dione der Formel Ia nach Anspruch 3, in der X¹ für eine durch ein Sauerstoff unterbrochene C₁-C₃-Alkylen- C₂-C₃-Alkenylen- oder C₂-C₃-Alkinylenkette steht.

5. 2-Benzoyl-cyclohexan-1,3-dione der Formel Ia nach Anspruch 3 und 4, in der Het für eine fünf- oder sechsgliedrige, teilweise oder vollständig gesättigte heterocyclische Gruppe, die ein Heteroatom ausgewählt aus einer Gruppe bestehend aus Sauerstoff, Stickstoff und Schwefel enthält, steht.

6. 4-Benzoyl-cyclohexan-1,3-dione gemäß Anspruch 1 der Formel 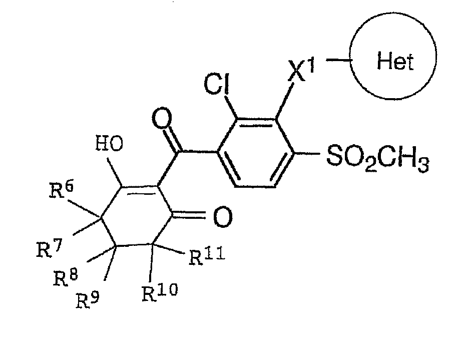 wobei die Symbole die in Anspruch 1 angegebenen Bedeutungen haben.

7. Verfahren zur Herstellung von 2-Benzoyl-cyclohexan-1,3-dionen der Formel I gemäß den Ansprüchen 1 bis 6, **dadurch gekennzeichnet, dass** man ein gegebenenfalls substituiertes Cyclohexan-1,3-dion Q mit einer aktivierten Carbonsäure IIIa oder mit einer Carbonsäure IIIb, 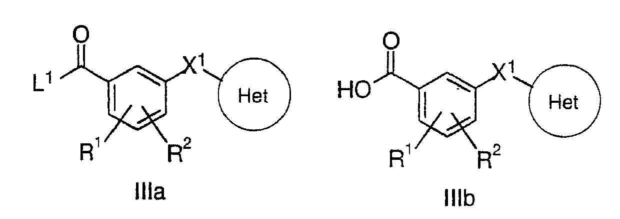 wobei die Substituenten R¹, R², X¹ und Het die in Anspruch 1 genannte Bedeutung haben und L¹ für eine nucleophil aus tauschbare Abgangsgruppe steht, acyliert und das Acylierungsprodukt gegebenenfalls in Gegenwart eines Katalysators zu den Verbindungen I umlagert.

8. Aktivierte Carbonsäuren der Formel IIIa gemäß Anspruch 7, wobei die Substituenten R¹, R², X¹ und Het die in Anspruch 1 genannte Bedeutung haben und L¹ für eine nucleophil austauschbare Abgangsgruppe steht gewählt aus Halogen, Imidazolyl, Pyridyl, Acetat und Trifluoracetat steht.

9. Mittel, enthaltend eine herbizid wirksame Menge mindestens eines 2-Benzoyl-cyclohexan-1,3-dions der Formel I oder eines landwirtschaftlich brauchbaren Salzes von I gemäß den Ansprüchen 1 bis 6 und für die Formulierung von Pflanzenschutzmitteln übliche Hilfsmittel.

10. Verfahren zur Herstellung von herbizid wirksamen Mitteln gemäß Anspruch 9, **dadurch gekennzeichnet, dass** man eine herbizid wirksame Menge mindestens eines 2-Benzoyl-cyclohexan-1,3-dions der Formel I oder eines landwirtschaftlich brauchbaren Salzes von I gemäß den Ansprüchen 1 bis 6 und für die Formulierung von Pflanzenschutzmitteln übliche Hilfsmittel mischt.

11. Verfahren zur Bekämpfung von unerwünschtem Pflanzenwuchs, **dadurch gekennzeichnet, dass** man eine herbizid wirksame Menge mindestens eines 2-Benzoyl-cyclohexan-1,3-dions der Formel I oder eines landwirtschaftlich brauchbaren Salzes von I gemäß den Ansprüchen 1 bis 6 auf Pflanzen, deren Lebensraum und/oder auf Samen einwirken lässt

12. Verwendung der 2-Benzoyl-cyclohexan-1,3-dione der Formel I und deren landwirtschaftlich brauchbaren Salze gemäß den Ansprüchen 1 bis 6 als Herbizide.
